(19) Europäisches Patentamt
European Patent Office
Office européen des brevets

(11) EP 0 465 048 B1

(12) EUROPEAN PATENT SPECIFICATION

(45) Date of publication and mention of the grant of the patent:
**11.09.1996 Bulletin 1996/37**

(51) Int Cl.6: **C07C 69/94**, C07C 69/773, C09K 19/32, G02F 1/13

(21) Application number: **91305546.3**

(22) Date of filing: **19.06.1991**

(54) **Carboxylic acid ester compound, liquid crystal compound, liquid crystal composition, liquid crystal element and light modulation method**

Carbonsäureesterverbindung, flüssigkristalline Verbindung, flüssigkristalline Zusammensetzung, flüssigkristallines Element und Methode zur Lichtmodulation

Composé ester d'acide carboxylique, composé cristal liquide, composition cristal liquide, élément cristal liquide et méthode de modulation de la lumière

(84) Designated Contracting States:
**AT BE CH DE DK ES FR GB GR IT LI LU NL SE**

(30) Priority: **25.06.1990 JP 166392/90**
**29.11.1990 JP 331873/90**
**29.11.1990 JP 331874/90**

(43) Date of publication of application:
**08.01.1992 Bulletin 1992/02**

(73) Proprietor: **MITSUI PETROCHEMICAL INDUSTRIES, LTD.**
**Tokyo 100 (JP)**

(72) Inventors:
- **Nishiyama, Shinichi, c/o Mitsui Petrochemical Sodegaura-shi, Chiva 29902 (JP)**
- **Hama, Hideo, c/o Mitsui Petrochemical Sodegaura-shi, Chiva 29902 (JP)**
- **Miyakoshi, Shoicho, c/o Mitsui Petrochemical Sodegaura-shi, Chiva 29902 (JP)**
- **Yamanaka, Tooru, c/o Mitsui Petrochemical Sodegaura-shi, Chiva 29902 (JP)**

(74) Representative: **Cresswell, Thomas Anthony et al**
**J.A. KEMP & CO.**
**14 South Square**
**Gray's Inn**
**London WC1R 5LX (GB)**

(56) References cited:

| | |
|---|---|
| EP-A- 0 332 409 | EP-A- 0 332 456 |
| EP-A- 0 339 987 | EP-A- 0 341 922 |
| EP-A- 0 401 961 | EP-A- 0 413 585 |
| EP-A- 0 422 996 | WO-A-87/06577 |
| JP-A- 3 106 850 | JP-A-62 010 045 |

EP 0 465 048 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

The present invention relates to novel carboxylic acid ester compounds, liquid crystal compounds, liquid crystal compositions, liquid crystal elements, light modulation methods using the liquid crystal elements and display methods utilizing the light modulation methods. More particularly, the invention relates to novel liquid crystal elements using liquid crystal materials containing anti-ferroelectric liquid crystals which contain the above-mentioned novel carboxylic acid ester compounds, light modulation methods using the novel liquid crystal elements and display methods utilizing the light modulation methods.

Most conventional display devices such as those used for office automation machines are driven by the TN (twisted nematic) mode.

When the TN mode is adopted, however, there are problems such that positions of molecules of a liquid crystal compound used in the display device must be changed in order to change an image being displayed, so that the time required for driving the device is prolonged. A high voltage is also required to change the positions of the molecules of the liquid crystal compound, that is, the power consumption is large.

On the other hand, switching elements using ferroelectric liquid crystal compounds are able to function only by changing the direction of molecular orientation of the liquid crystal compounds, and hence the switching time required for operating the switching elements is markedly shortened. Further, a value of Ps x E obtained from a spontaneous polarization (Ps) of the ferroelectric liquid crystal compound and an intensity of the electric field (E) applied is an effective energy output for changing the direction of molecular orientation of the liquid crystal compound, so that power consumption required therefor can also be markedly minimized. Such ferroelectric liquid crystal compounds as mentioned above are particularly suitable for use in display devices for moving pictures, because they have two steady states (i. e., bi-stability) depending upon the direction of electric field applied. They have excellent switching threshold value characteristics.

When these ferroelectric liquid crystal compounds are intended for use in optical switching elements, they are required to have various characteristics such as an operating temperature in the vicinity of ordinary temperature or below, a wide operating temperature zone, a high switching speed and an appropriate switching threshold value voltage.

Known ferroelectric liquid crystal compounds generally have a narrow operating temperature range. Even in ferroelectric liquid crystal compounds having a wide operating temperature range, other characteristics are insufficient, as disclosed in R.B. Meyer et al., J. de Phys., Vol. 36 L, p. 69 (1975) and the paper reported by Masaaki Taguchi and Takamasa Harada, "Proceedings of Eleventh Conference on Liquid Crystal", p. 168 (1985). Thus, no ferroelectric liquid crystal compounds which are satisfactory for practical use are yet available.

There have also been various proposals for light modulation elements using ferroelectric liquid crystals.

For driving such light modulation elements, there is known a method of using a liquid crystal cell composed of two transparent substrates being arranged so as to face each other, leaving a gap of about 2 μm between the substrates, the gap being filled with a ferroelectric liquid crystal in a chiral smectic C phase.

The ferroelectric liquid crystal has a layer structure in the chiral smectic C phase, and in this layer, the major axis of the liquid crystal molecule is oriented to have a practically definite angle θ (called a tilt angle). In this state, as shown in Fig. 1, the major axis of the liquid crystal molecule 11 gradually turns to a different direction owing to an interaction between the molecules and thereby forms a helical structure (Fig. 1).

However, when a gap of about 2 μm formed by two glass substrates is filled with a liquid crystal material, the oriented state of the liquid crystal material is influenced by the glass substrates to release its helical structure, and the liquid crystal molecule 21 comes to exhibit two forms of steady state when viewed from above the transparent substrate 20 as shown in Fig. 2. In this steady state, the major axis of the liquid crystal molecule and a dipole perpendicular thereto take directions opposite to each other in the two forms of steady state, so that the steady state of the liquid crystal material can be transferred between the above-mentioned two steady states by applying an electric field thereto.

In that case, the amount of transmitted light can be controlled by arranging the above-mentioned liquid crystal cell between two polarizing plates whose polarization directions are at right angles so that the cell becomes dark (the amount of transmitted light decreases) when the liquid crystal material in the cell takes one form of the two forms of the steady state.

In this method, it is theoretically said that the steady state of the liquid crystal molecule involves only two forms as mentioned above. Therefore, it is said that when the liquid crystal material in the cell is once brought to the steady state by application of an electric field thereto, the liquid crystal material will not be transferred to another form of the steady state even when the electric field is eliminated. Accordingly a light modulation element using the above-mentioned liquid crystal cell is said to have a memory effect.

Actually, however, when the liquid crystal material held in a steady state is allowed to stand, as it is, without application thereto of an electric field, a part of the liquid crystal material is sometimes transferred to the other form of the steady state. It is difficult to impart a sufficient memory effect to the light modulation element. In other words, it is difficult to maintain the liquid crystal material in a definite steady state for a long period of time without application

thereto of an electric field. Therefore, in order to maintain the steady state of the liquid crystal material, namely, a bright state and a dark state of the light modulation element, it is necessary to apply an electric field of a certain degree thereto.

In the above-mentioned conventional method, the application of an electric field is required even in the case of attaining a dark state. In most cases it is difficult to attain a dark state having a sufficient darkness. On that account, it has not been possible to obtain a sufficient ratio between the bright state and the dark state, namely, a sufficiently high contrast.

EP-A-341,922 describes naphthalene compounds which can be used in liquid crystal compositions. These compounds have only a single divalent phenyl group and do not have a smectic C phase.

EP-A-332,456 discloses liquid crystal compounds used in liquid crystal elements. These compounds have a smectic A phase.

EP-A-339,987 discloses liquid crystal compounds having a naphthalene nucleus. These compounds are stated to have a smectic C phase, but the temperature range thereof is very narrow.

JP-A-62/010,045 discloses liquid crystal compounds having a naphthalene nucleus. Again although these compounds have a smectic C phase, the operating range thereof is relatively narrow.

WO-A-87/06577 discloses liquid crystal compounds containing a naphthalene nucleus. The pure compounds do not have a smectic C phase.

EP-A-332,409 discloses liquid crystal compounds having a naphthalene nucleus. It is specifically indicated that some compounds do not have a smectic C phase. In any case the operating temperature range is narrow.

The present invention seeks to provide liquid crystal compounds and liquid crystal compositions, both capable of forming display devices having excellent characteristics such as an operating temperature of a wide range, high switching speed, switching threshold value voltage of an appropriate range and operability with very small power consumption.

The present invention has been made in view of the prior art wherein light modulation methods and light modulation elements having sufficiently high contrast have not yet been obtained. Accordingly the present invention also seeks to provide liquid crystal elements capable of attaining a dark state of sufficient darkness and having a high contrast, and light modulation methods using such liquid crystal elements.

The present invention provides a first carboxylic acid ester compound represented by formula (I):

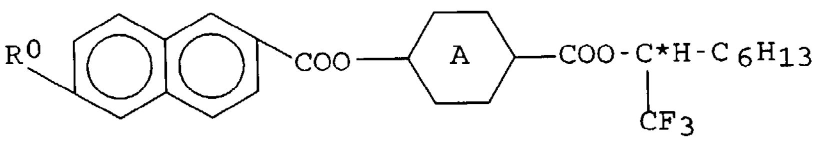

(I)

wherein $R^0$ is alkyl group of 3-20 carbon atoms, alkoxy group of 3-20 carbon atoms or halogenated alkyl group of 3-20 carbon atoms; C* is an asymmetric carbon atom; and

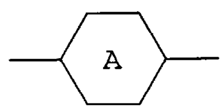

represents a group selected from

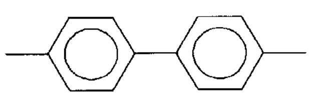 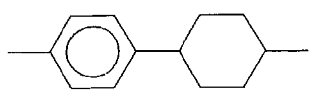 and 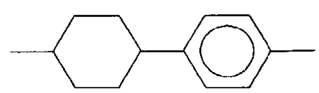 .

The present invention also provides a second carboxylic acid ester compound represented by formula (II):

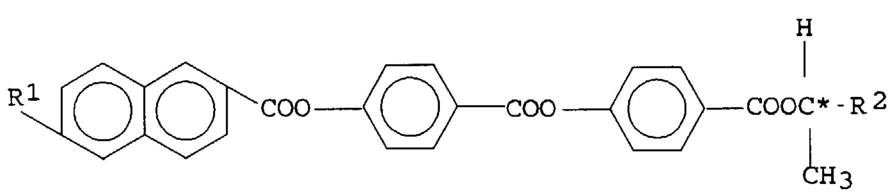

(II)

wherein $R^1$ is an alkyl group of 3-20 carbon atoms, alkoxy group of 3-20 carbon atoms or halogenated alkyl group of 3-20 carbon atoms; C* is an asymmetric carbon atom; and $R^2$ is an alkyl group of 2-10 carbon atoms.

The carboxylic acid ester compounds of formulae (I) and (II) have optical activity.

The present invention also provides the use of a compound of formula (I) or (II) as a liquid crystal compound.

The present invention additionally provides a liquid crystal composition comprising a compound of formula (I) or (II).

Employment of the carboxylic acid ester compounds of the invention as liquid crystal compounds makes it possible

to produce various display devices having excellent characteristics such as an operating temperature of a wide range, a high switching speed, operability with a very small power consumption and a stable contrast.

The present invention further provides a liquid crystal element comprising a cell and two polarizing plates, one on each outer side of the cell, said cell being composed of two transparent substrates so arranged as to face each other leaving a gap therebetween and a transparent electrode provided on the inner surface of each substrate, said gap being filled with a liquid crystal material, wherein:

said two polarizing plates are so arranged that an angle formed by polarization planes of the polarizing plates is in the range of 70°-110°, and said cell filled with the liquid crystal material is arranged between the polarizing plates at an angle of from +10° to -10° relative to the position of the cell at which the transmitted light becomes the darkest or the brightest;
and
the liquid crystal material comprising a liquid crystal compound represented by formula (III):

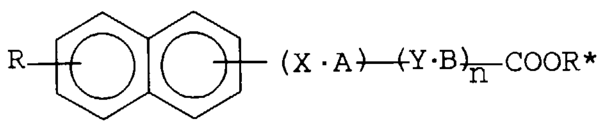 (III)

wherein R is an alkyl group of 3-20 carbon atoms, alkoxy group of 3-20 carbon atoms or halogenated alkyl group of 3-20 carbon atoms; X and Y each represent a group selected from -COO-, -OCO-, $-CH_2CH_2-$, $-CH_2O-$, $OCH_2-$,

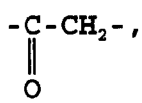, 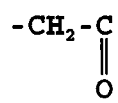,

and -S-S- or a single bond;
R* represents a group selected from

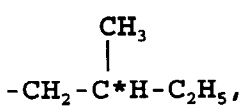, 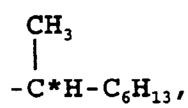, 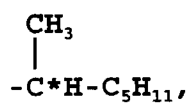, 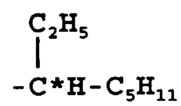,

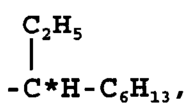, 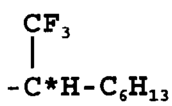 and 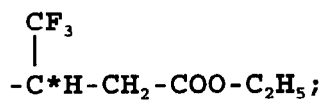;

n is an integer of 1 or 0; and when n=0, A is

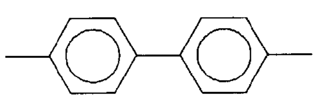 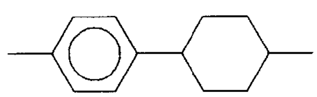 or 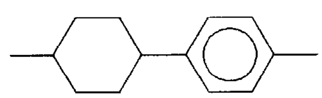

and when n=1, A and B each is

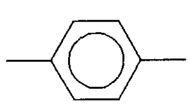 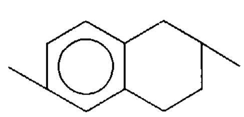 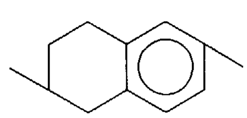 or 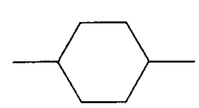 .

The liquid crystal compound of formula (II) has optical activity.

The present invention also provides a light modulation method which comprises applying an electric field to a liquid crystal element as defined above.

The display element and the display method according to the invention are characterized in that they use the abovementioned liquid crystal element.

According to the liquid crystal element of the invention and the light modulation method of the invention, a dark state having a sufficient darkness can be obtained, so that not only the contrast between a bright state and a dark state can be prominently enhanced, but also excellent memory effects can be obtained.

The present invention additionally provides a display element using a liquid crystal element as defined above, and a display method utilizing a light modulation method as defined above.

Fig. 1 is a view illustrating that a major axis of each molecule of a ferroelectric liquid crystal tilts by a tilt angle to the vertical direction Z of a smectic phase, and the direction of the tilt rotates every smectic phase by a definite angle one by one, thereby forming a helical structure.

Fig. 2 is a view illustrating the state of a conventional thin film element wherein two types of the orientation direction can be attained.

Fig. 3 is a chart showing the $^1$H-NMR spectrum of the 1'''-trifluoromethylheptyl ester of trans-4-[4'-(6'''-n-decyloxy-2''-naphthoyloxy)phenyl]cyclohexane carboxylic acid.

Fig. 4 is a sectional view schematically showing one embodiment of the liquid crystal element of the present invention.

Figs. 5 and 6 are each photographs of oscillographic waves showing the relationship between voltage and intensity of transmitted light when applying a triangular wave to the liquid crystal element of the present invention in which a liquid crystal cell is so arranged as to attain the darkest state.

Figs. 7 and 8 are photographs of oscillographic waves showing the relationship between voltage and intensity of transmitted light when applying a triangular wave to the liquid crystal element of the present invention in which a liquid crystal cell is so arranged as to attain the brightest state.

In formula [III], when R is an alkyl group having 3-20 carbon atoms, the alkyl group may be a straight-chain, branched or alicyclic group. However, the carboxylic esters in which R is a straight-chain alkyl group exhibit excellent liquid crystal properties, because their molecules take a rigid structure extending in a straight line. Examples of straight-chain alkyl groups are a hexyl group, a heptyl group, an octyl group, a decyl group, a dodecyl group, a hexadecyl group and an octadecyl group.

When R is a halogenated alkyl group having 3-20 carbon atoms, examples of such groups are groups obtained by substituting at least a part of the hydrogen atoms of the above-mentioned alkyl groups with a halogen atom such as F, Cl, Br or I.

When R is an alkoxy group having 3-20 carbon atoms, examples of such groups are alkoxy groups having the above-mentioned alkyl groups, for example, a hexoxy group, a heptoxy group, an octyloxy group, a decyloxy group, a dodecyloxy group, a tetradecyloxy group, a heptadecyloxy group, a hexadecyloxy group and an octadecyloxy group.

Compounds of formula [III] in which R is an alkoxy group exhibit particularly excellent liquid crystal properties.

In formula [III], in consideration of the linearity of the molecule, it is desired that any one of X and Y is -COO-, preferably that both X and Y are -COO-.

When using the carboxylic acid ester compound of formula [III] as a liquid crystal compound, it is preferred that A and B are

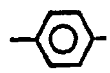

in consideration of the properties required for liquid crystal materials.

In formula [III] R* is preferably the following group in consideration of the properties required for liquid crystal materials:

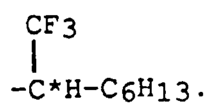

Examples of the naphthyl group in formula [III] are a 1,5-naphthyl group, a 1,6-naphthyl group, a 2,6-naphthyl group and a 1,7-naphthyl group.

In the present invention, the whole molecule is preferably linear, so that a 2,6-naphthyl group is particularly preferred as the naphthyl group.

Preferred compounds of formula [III] are those of the following formulae [301] to [304].

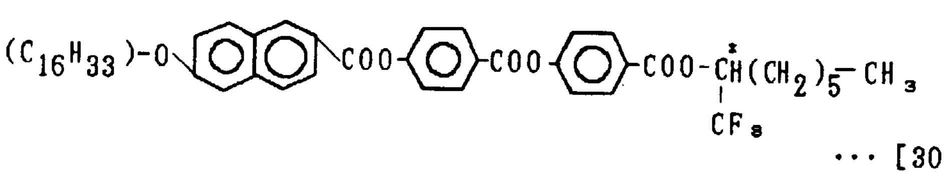

··· [301]

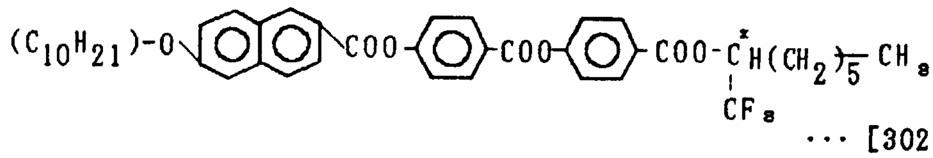

... [302]

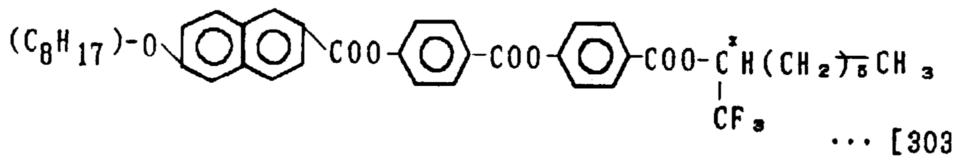

... [303]

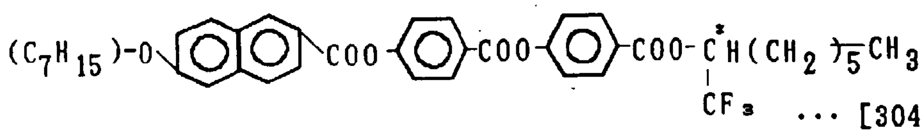

... [304]

The above-mentioned carboxylic acid ester compounds can be prepared by utilizing a combination of known synthesis techniques.

For example, the carboxylic acid ester compounds can be synthesized through a synthesis route as illustrated below:

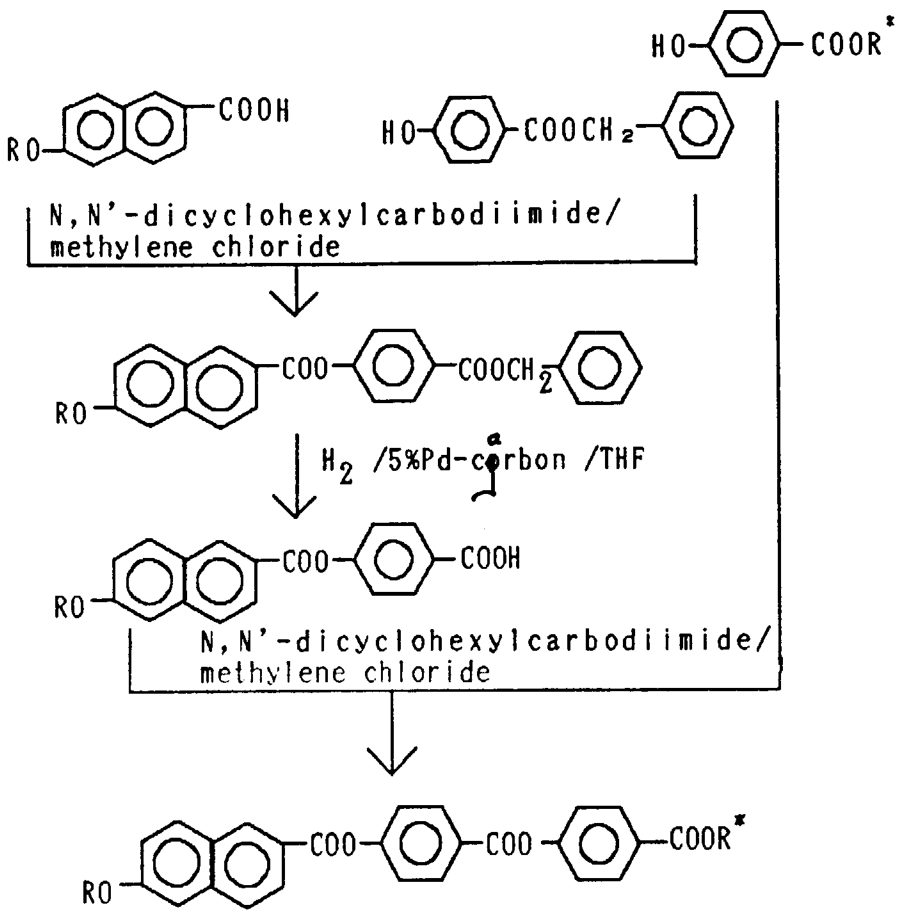


That is, the benzyl ester of 4-(6'-n-alkoxy-2'-naphthoyloxy)benzoic acid is obtained, for example, by reacting 6-n-alkoxynaphthalene-2-carboxylic acid with 4-hydroxybenzoic acid benzyl ester in the presence of 4-N,N-dimethylaminopyridine using methylene chloride as a solvent while adding dropwise a methylene chloride solution containing N,N'-dicyclohexylcarbodiimide.

The thus obtained 4-(6'-n-alkoxy-2'-naphthoyloxy)benzoic acid benzyl ester is introduced into tetrahydrofuran, and reduced with hydrogen gas in the presence of a reduction catalyst such as a palladium/carbon catalyst to obtain 4-(6'-n-alkoxy-2'-naphthoyloxy)benzoic acid.

Subsequently, an ester obtained from hydroxybenzoic acid and an alcohol having an asymmetric carbon atom is reacted with the 4-(6'-n-alkoxy-2'-naphthoxyloxy)benzoic acid obtained in the above step using methylene chloride as a solvent in the presence of 4-N,N'-dimethylaminopyridine while adding dropwise a methylene chloride solution containing N,N'-dicyclohexylcarbodiimide, to obtain a carboxylic acid ester compound employable in the invention.

The above-mentioned process is given as an example of a process for preparing the carboxylic acid ester compounds employable in the invention. It should be construed that processes for preparing the carboxylic acid ester compounds employable in the invention are not limited to the above-mentioned process.

Of the carboxylic acid ester compounds of formula [III] obtained as above, a compound of formula [304] exhibits particularly excellent liquid crystal properties.

The phase transition temperatures of the compound of formula [304] are set forth in Table 1. In the present invention, Cry represents a crystal phase, SmC* represents a chiral smectic phase, SmA represents a smectic A phase, and Iso represents an isotropic liquid.

Table 1

| | Phase transition temperature | | |
|---|---|---|---|
| Compound | Cry-SmC* | SmC*-SmA | SmA-Iso |
| [304] | 73 °C | 125 °C | 183 °C |

Among the liquid crystal compounds of formula [III], many compounds have a smectic phase within a wide temperature range as shown in Table 1.

In the present invention, the compounds of formula [III] are employed as the liquid crystal compounds. The compounds of formula [III] include novel carboxylic acid ester compounds of formula [I].

In formula [I], when $R^0$ is an alkyl group having 3-20 carbon atoms, the alkyl group may be a straight-chain, branched or alicyclic group. However, the carboxylic esters in which R is a straight-chain alkyl group exhibit excellent liquid crystal properties, because their molecules take a rigid structure extending in a straight line. Preferred straight-chain alkyl groups are groups having 6-20 carbon atoms. Examples of straight-chain alkyl groups are a hexyl group, a heptyl group, an octyl group, a decyl group, a dodecyl group, a tetradecyl group, a hexadecyl group and an octadecyl group.

When $R^0$ is a halogenated alkyl group having 3-20 carbon atoms, examples of such groups are groups obtained by substituting at least a part of the hydrogen atoms of the above-mentioned alkyl groups with a halogen atom such as F, Cl, Br or I.

When $R^0$ is an alkoxy group having 3-20 carbon atoms, examples of such groups are alkoxy groups having the above-mentioned alkyl groups, for example, a hexoxy group, a heptoxy group, an octyloxy group, a decyloxy group, a dodecyloxy group, a tetradecyloxy group, a heptadecyloxy group, a hexadecyloxy group and an octadecyloxy group.

When using the carboxylic acid ester compounds of formula [I] as liquid crystal compounds, useful compounds are those wherein $R^0$ is an alkoxy group.

When using the carboxylic acid ester compounds of formula [I] as liquid crystal compounds,

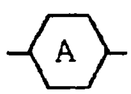

preferably is any one of

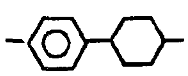

and

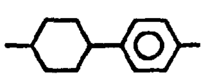

in consideration of the properties required for liquid crystal materials.

Preferred carboxylic acid ester compounds of formula [I] are those having the following formulae [113] to [124] and [137] to [148].

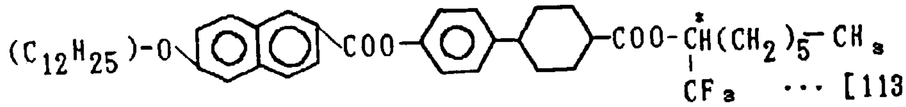
[113]

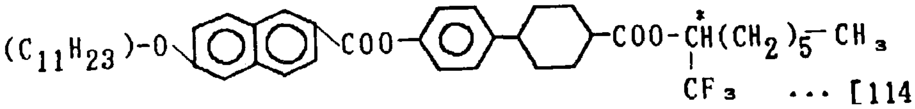
[114]

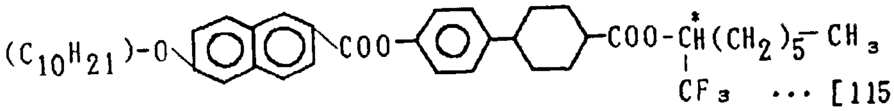
[115]

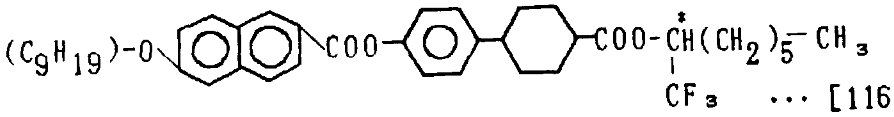
[116]

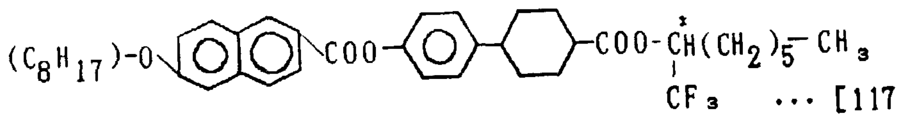
[117]

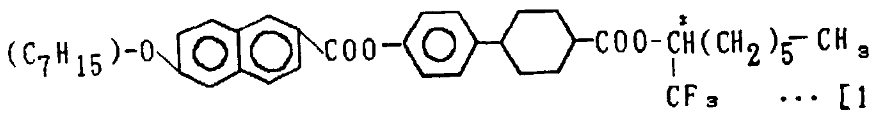
[118]

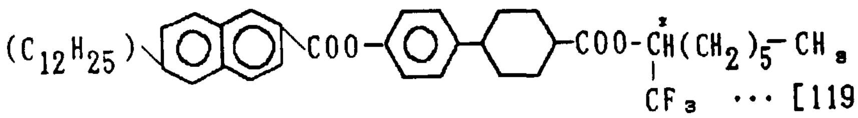
[119]

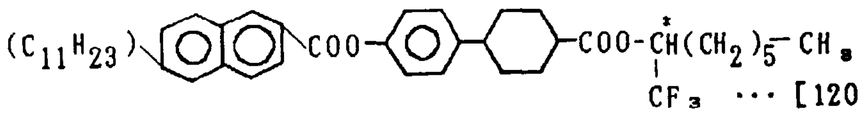
[120]

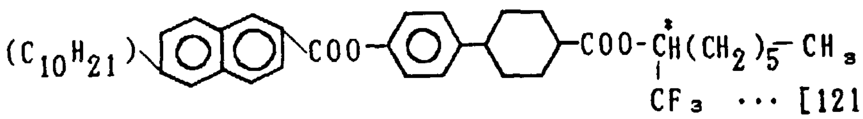
[121]

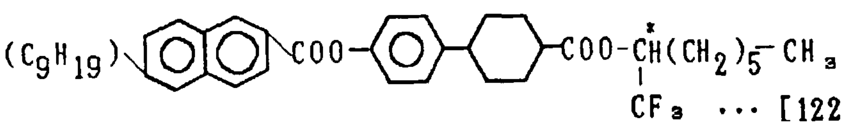
[122]

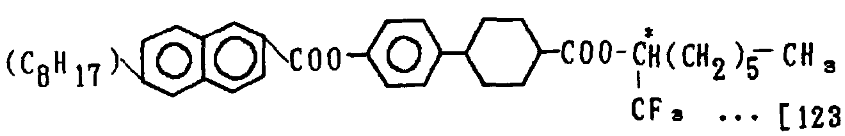
[123]

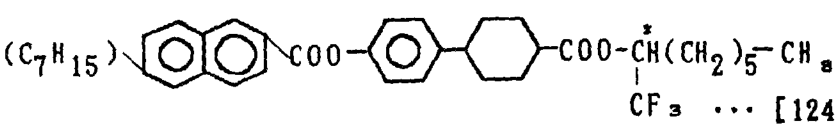
[124]

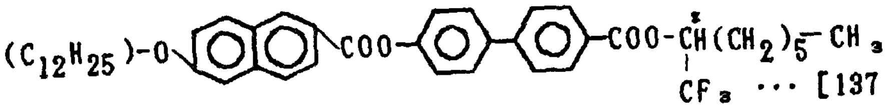
[137]

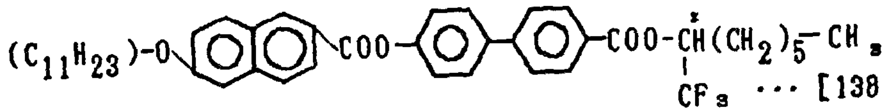
[138]

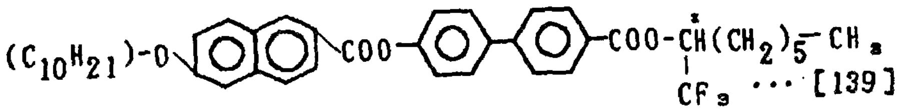
[139]

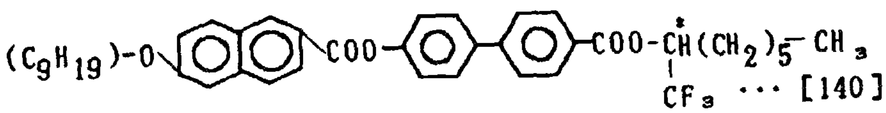
[140]

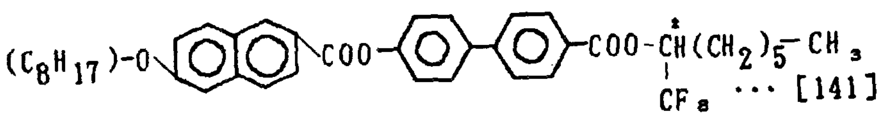
[141]

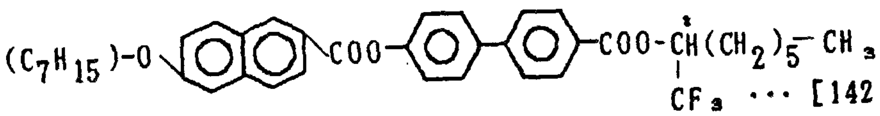
[142]

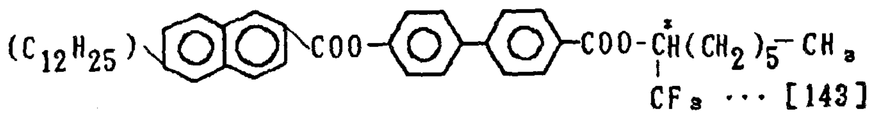
[143]

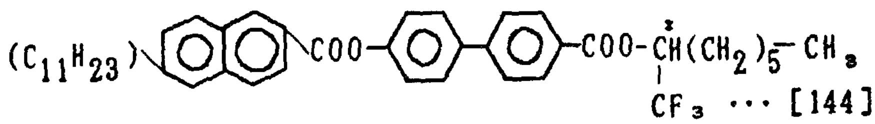
[144]

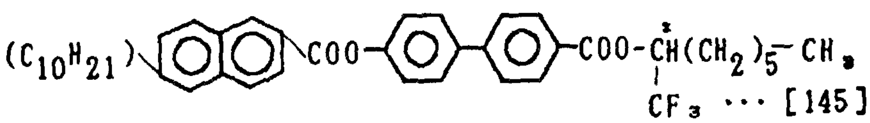
[145]

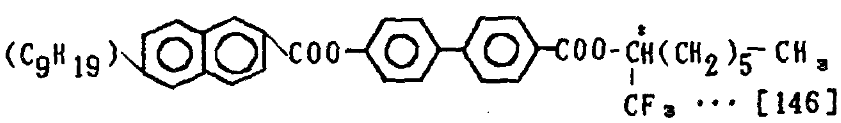
[146]

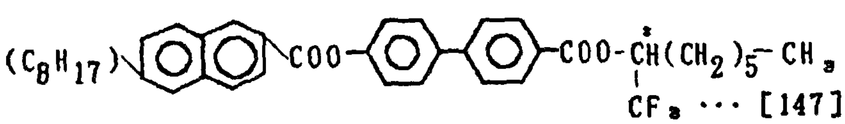
[147]

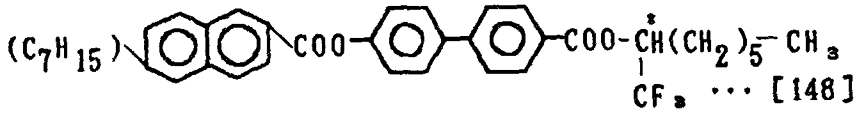

... [148]

The above-mentioned first carboxylic acid ester compound of the invention can be prepared by utilizing a combination of known synthesis techniques.

For example, the first carboxylic acid ester compound can be synthesized through the following synthesis route. In the following synthesis route, the synthesis of the first carboxylic acid ester compound is illustrated by exemplifying a carboxylic acid ester compound having the formula [I] wherein $R^0$ is an alkoxy group.

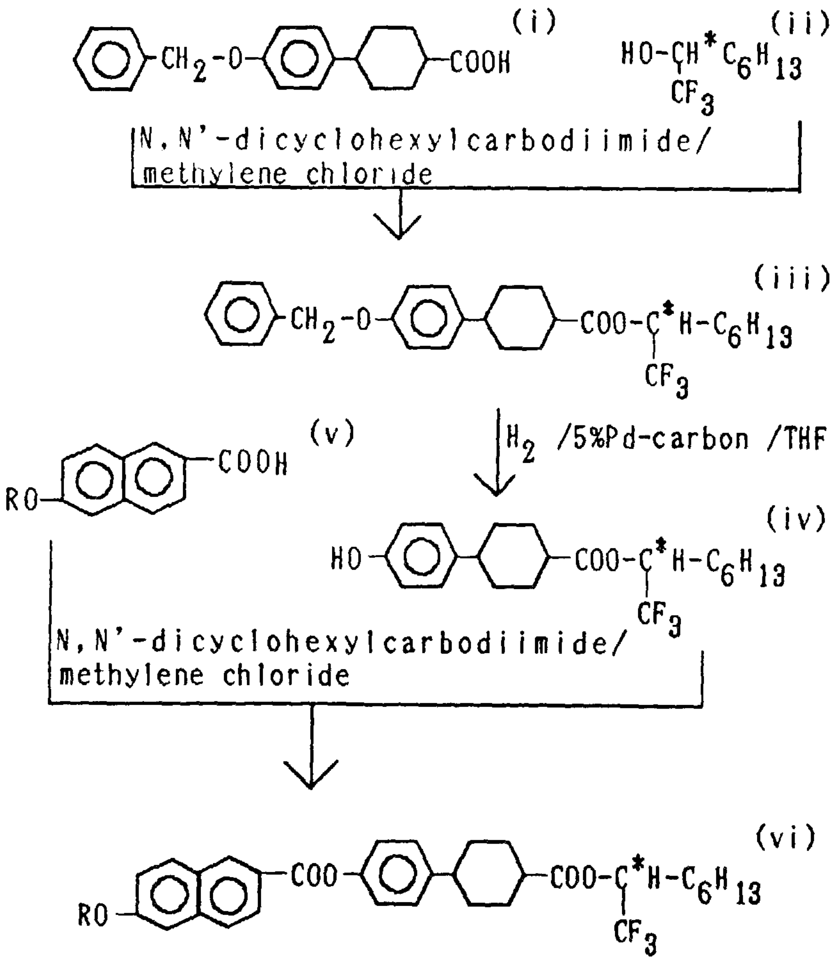


That is, the 1"-trifluoromethylheptyl ester of trans-4-(4'-benzyloxyphenyl)cyclohexane carboxylic acid (iii) is obtained, for example, by reacting trans-4-(4'-benzyloxyphenyl)cyclohexane carboxylic acid (i) with 1-trifluoromethylheptyl alcohol (ii) using a mixture of 4-N,N-dimethylaminopyridine and methylene chloride as a solvent while adding dropwise an esterifying agent such as N,N'-dicyclohexycarbodiimide.

The thus obtained 1"-trifluoromethylheptyl ester of trans-4-(4'-benzyloxyphenyl)cyclohexane carboxylic acid (iii) is introduced into a solvent such as tetrahydrofuran, and reduced with hydrogen gas in the presence of a reduction catalyst such as a palladium/carbon catalyst, to obtain the 1"-trifluoromethylheptyl ester of trans-4-(4'-hydroxyphenyl) cyclohexane carboxylic acid (iv).

Subsequently, 6-alkoxynaphthalene-2-carboxylic acid (v) is reacted with the 1"-trifluoromethylheptyl ester of trans-4-(4'-hydroxyphenyl)cyclohexane carboxylic acid (iv) obtained in the above step using a mixture of 4-N,N-dimethylaminopyridine and methylene chloride as a solvent while adding dropwise N,N'-dicyclohexanecarbodiimide to obtain a carboxylic acid ester compound of formula [I].

The above-mentioned process is given as an example of a process for preparing the carboxylic acid ester compounds of formula [I]. It should be construed that processes for preparing the carboxylic acid ester compounds are not limited to the above-mentioned process.

Fig. 3 is a $^1$H-NMR spectrum chart of the 1'''-trifluoromethylheptyl ester of trans-4-[4'-(6"-n-decyloxy-2"-naphthoyloxy)phenyl]cyclohexane carboxylic acid having the following formula selected, for example, out of the carboxylic acid ester compounds of formula [I] prepared by the above-mentioned process.

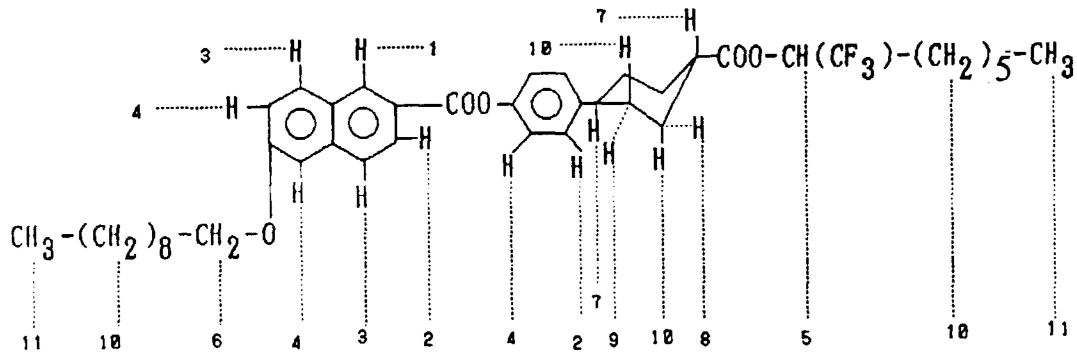

In the above-mentioned formula, numerals 1 to 11 indicate the numbers of hydrogen atoms, and the numbers correspond to numbers attached to the peaks shown in Fig. 3.

The carboxylic acid ester compounds of formula [I] prepared by the above-mentioned process can be favorably employed as liquid crystal compounds.

Of the carboxylic acid ester compounds mentioned above, those of formulae [115] and [139] exhibit particularly excellent liquid crystal properties.

The phase transition temperatures of the liquid crystal compounds of formulae [115], and [139] are set forth in Table 2.

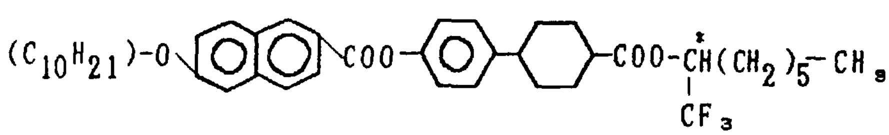

[115]

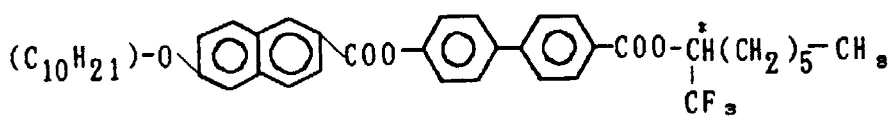

[139]

Table 2

| | Phase transition temperature | | |
|---|---|---|---|
| Compound | Cry-SmC* or SmA | SmC*-SmA | SmA-Iso |
| [115] | 40 °C | 90 °C | 116 °C |
| [139] | 25 °C | 92 °C | 123 °C |

Compounds [115] and [139] illustrate the present invention.

Compound [127] is for comparison.

Among the carboxylic acid ester compounds (i.e., liquid crystal compounds) of formula [I], many compounds have a smectic phase over a wide temperature range as shown in Table 2.

The carboxylic acid ester compounds of formula [III] also include novel carboxylic acid ester compounds of formula [II].

In formula [II], when $R^1$ is an alkyl group having 3-20 carbon atoms, the alkyl group may be a straight-chain, branched or alicyclic group. The carboxylic esters in which $R^1$ is a straight-chain alkyl group exhibit excellent liquid crystal properties, because their molecules take a rigid structure extending in a straight line. Preferred straight-chain alkyl groups are straight-chain alkyl groups having 6-20 carbon atoms. Examples of such groups are a hexyl group, a heptyl group, an octyl group, a nonyl group, a decyl group, a undecyl group, a dodecyl group, a tetradecyl group, a hexadecyl group and an octadecyl group.

When $R^1$ is a halogenated alkyl group, examples of such groups are groups obtained by substituting at least a

part of hydrogen atoms of the above-mentioned alkyl groups with a halogen atom such as F, Cl, Br or I.

When $R^1$ is an alkoxy group, examples of such groups are alkoxy groups having the above-mentioned alkyl groups, for example, a hexoxy group, a heptoxy group, an octyloxy group, a nonyloxy group, a decyloxy group, a undecyloxy group, a dodecyloxy group, a tetradecyloxy group, a heptadecyloxy group, a hexadecyloxy group and an octadecyloxy group.

When using the carboxylic acid ester compounds of formula [II] as liquid crystal compounds, those therein $R^1$ is an alkoxy group, particularly an alkoxy group having 7-16 carbon atoms, are useful.

In formula [II], $R^2$ is preferably an alkyl group having 2-6 carbon atoms. Examples of the group $R^2$ are ethyl group, a propyl group, a butyl group, a pentyl group, a hexyl group, a heptyl group and an octyl group. Of the compounds having those alkyl groups, particularly useful as liquid crystal compounds are compounds having a hexyl group ($-C_6H_{13}$).

The group $R^2$ is linked to an asymmetric carbon atom C*, and to the asymmetric carbon atom are linked a hydrogen atom and a methyl group ($-CH_3$). Further, to the residual linkage portion of the asymmetric carbon atom is linked a p-phenylene group (first phenylene group) through an ester linkage.

The p-phenylene group is linked to the other p-phenylene group (second phenylene group) through an ester linkage.

Furthermore, this phenylene group is linked to the 2,6-naphthalene group through an ester linkage.

When using the carboxylic acid ester compounds of the invention as liquid crystal compounds, the whole molecule preferably becomes linear. Thus particularly excellent liquid crystal compounds are the compounds of the invention wherein two p-phenylene groups are linked to a 2,6-naphthalene group through ester linkages.

To the other linkage portion of this naphthalene group is linked the above-mentioned $R^1$.

Preferred examples of the carboxylic acid ester compounds of formula [II] are those of formulae [201] to [216].

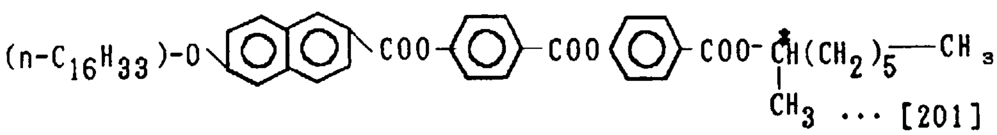
... [201]

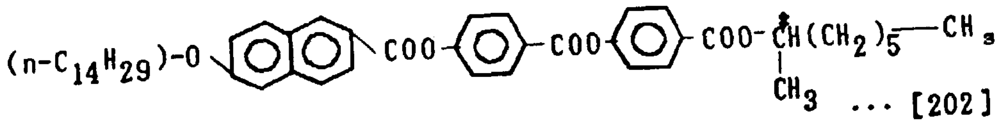
... [202]

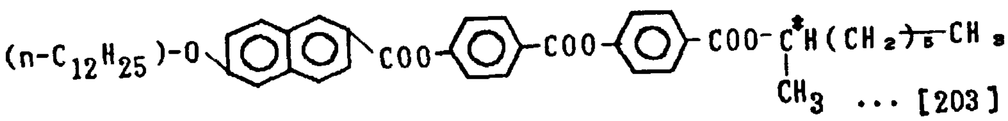
... [203]

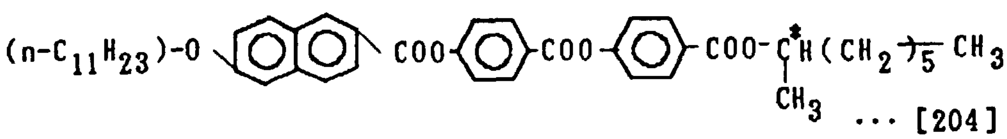
... [204]

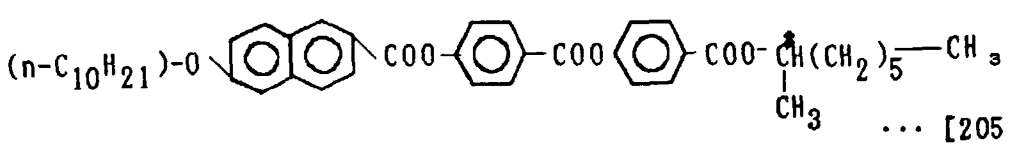
... [205]

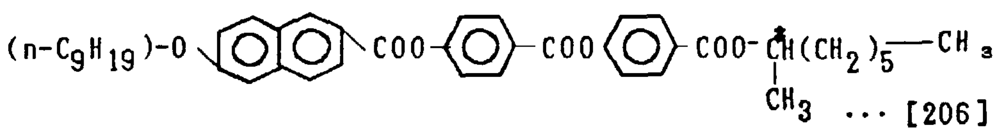
... [206]

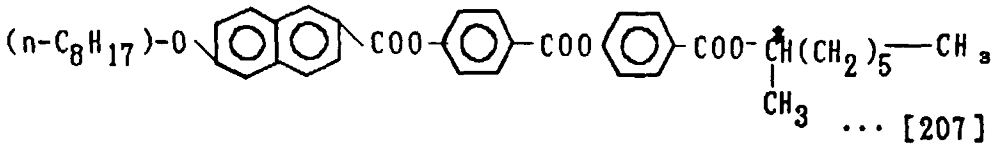

... [207]

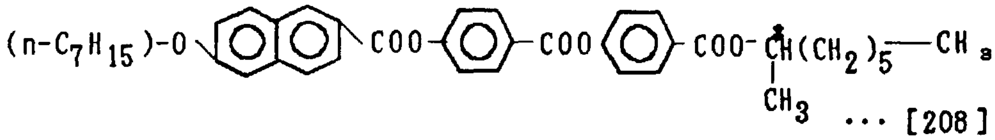

... [208]

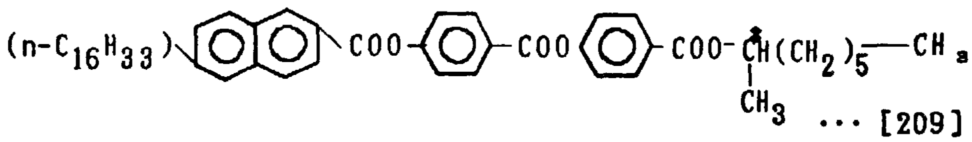

... [209]

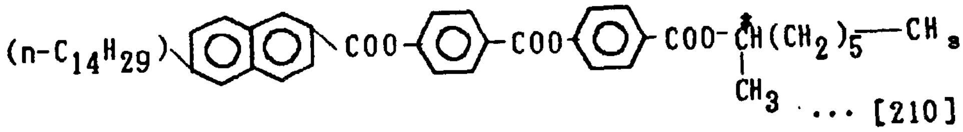

... [210]

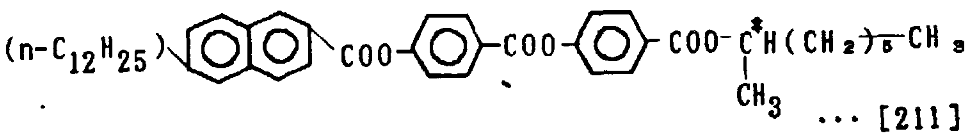

... [211]

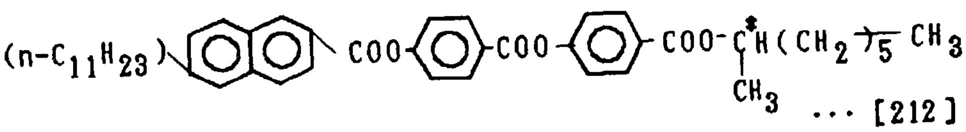

... [212]

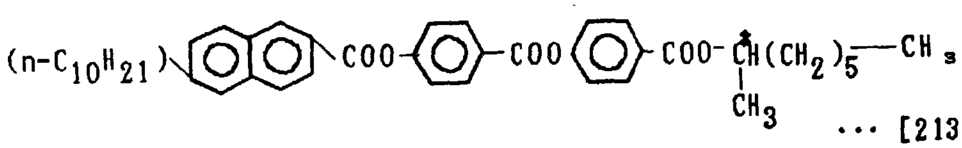

... [213]

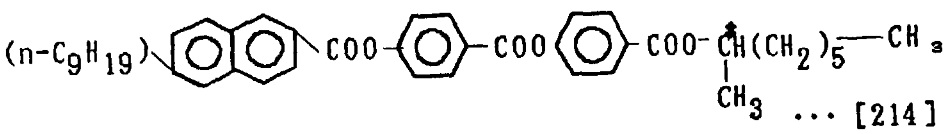

... [214]

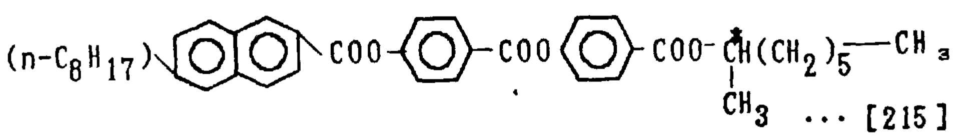

... [215]

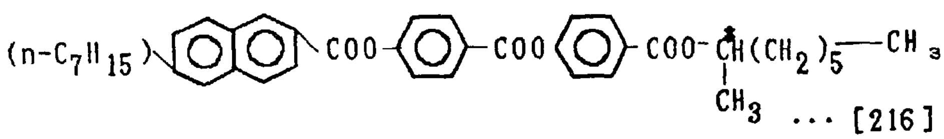

... [216]

The carboxylic acid ester compounds of formula [II] can be also prepared by utilizing a combination of known synthesis techniques, as described in the preparation of the carboxylic acid ester compounds of formula [III].

The carboxylic acid ester compounds of formula [II] can be preferably employed as the liquid crystal compounds.

Of such carboxylic acid ester compounds, those having the formula [II] wherein $R^1$ is an alkoxy group having 6-18 carbon atoms and $R^2$ is a hexyl group exhibit excellent liquid crystal properties. In particular, the compound having the formula [205] exhibits excellent liquid crystal properties.

The phase transition temperatures of the liquid crystal compound of formula [205] are set forth in Table 3.

Table 3

| | Phase transition temperature | | |
|---|---|---|---|
| Compound | Cry-SmC* or SmA | SmC*-SmA | SmA-Iso |
| [205] | 100 °C | 137 °C | 181 °C |

Among the carboxylic acid ester compounds (i.e., liquid crystal compounds) of formula [II], many compounds have a smectic phase over a wide temperature range as shown in Table 3.

When liquid crystal compounds are used singly as in the conventional cases, liquid crystal compounds having a smectic phase within a wide temperature range of as wide as 100 °C as in the case of the above-mentioned compound are hardly known.

For the liquid crystal elements, there have been conventionally employed liquid crystal materials having any one of a chiral smectic C phase, a chiral smectic F phase, a chiral smectic G phase, a chiral smectic H phase, a chiral smectic I phase, a chiral smectic J phase and a chiral smectic K phase. However, even if the liquid crystal materials other than the liquid crystal materials having a chiral smectic C phase are employed, the resulting liquid crystal elements have a slow response speed. Accordingly, driving with a chiral smectic C phase having a quick response speed has heretofore been considered to be advantageous in practical use.

However, the liquid crystal elements can be employed not only in the chiral smectic C phase but also in the chiral smectic A phase by utilizing a method of driving liquid crystal elements in the smectic A phase as proposed previously by the present inventors (see Japanese Patent Application No. 64-3632 (1989). Therefore, when the liquid crystal compounds containing the above-mentioned carboxylic acid ester compounds are employed, the obtained liquid crystal elements can be driven over a wide temperature range, and moreover, the liquid crystal elements filled with liquid crystal materials containing such liquid crystal compounds have excellent high-speed response.

The liquid crystal materials as mentioned above can be used singly or in combination with other liquid crystal compounds. For example, the liquid crystal compounds of the invention can be used as a principal ingredient in a smectic liquid crystal composition, or can be used as a minor ingredient in a liquid crystal composition containing another compound having a smectic phase as a principal ingredient. The content of the above-mentioned carboxylic acid ester compound (i.e. liquid crystal compound) in the liquid crystal material constituting the liquid crystal element of the invention can be appropriately determined in consideration of the characteristics of, for example, the liquid crystal compound used, the viscosity of the composition, the operating temperature of the resulting liquid crystal element, and the use application. In the invention, the content of the carboxylic acid ester compound (i.e. liquid crystal compound) in the liquid crystal material is generally 1-99 parts by weight, preferably 5-75 parts by weight. Further, in the liquid crystal material employable in the invention, the above-described carboxylic acid ester compounds may be contained alone or in combination of two or more compounds.

As liquid crystal compounds which can be used together with the carboxylic acid ester compounds of formulae [I], [II] or [III] in the liquid crystal material employable in the the invention, there can be mentioned, for example, (+)-4'-(2"-methylbutyloxy)phenyl-6-octyloxynaphthalene-2-carboxylic ester, 4'-decyloxyphenyl-6-((+)-2"-methylbutyloxy)naphthalene-2-carboxylic ester,

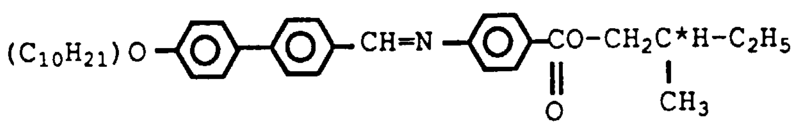

,

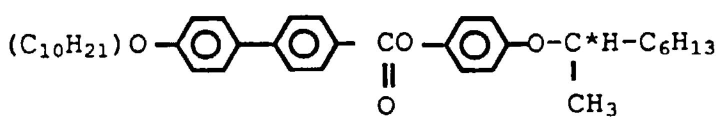

,

and

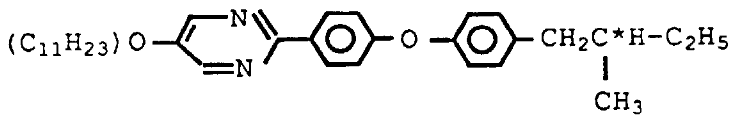 ,

Also employable are for example the following compounds having a cyclic structure:

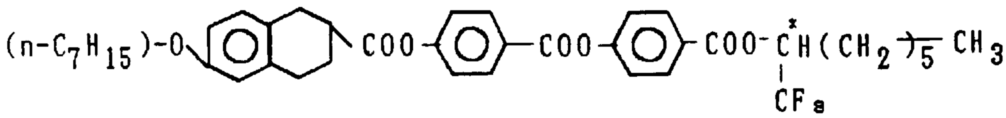

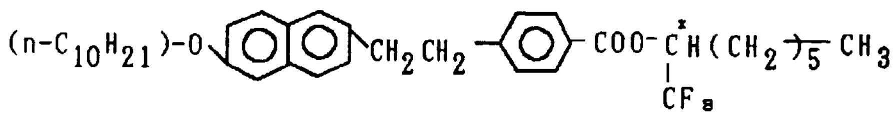

Compounds having an asymmetric carbon atom such as the following compound can be also employed:

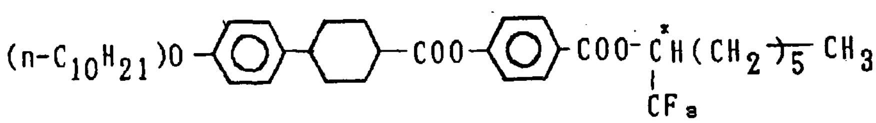

Moreover, also employable are: Shiff base type liquid crystal compounds such as

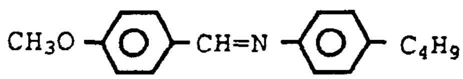

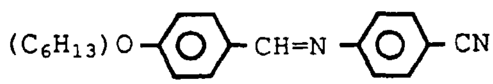

azoxy type liquid crystal compounds such as

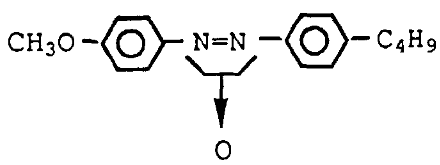

benzoic acid ester type liquid crystal compounds such as

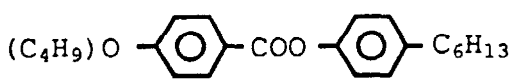

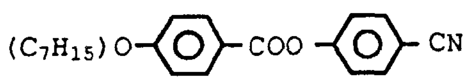

cyclohexylcarboxylic acid ester type liquid crystal compounds such as

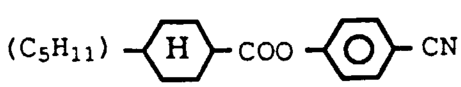

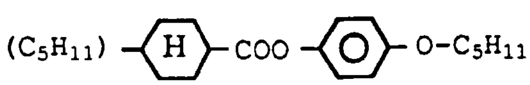

phenyl type liquid crystal compounds such as

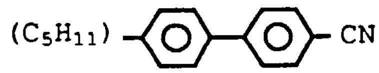

terphenol type liquid crystal compounds such as

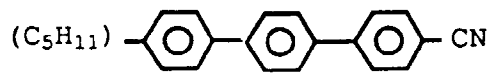

cyclohexyl type liquid crystal compounds such as

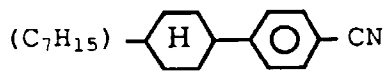

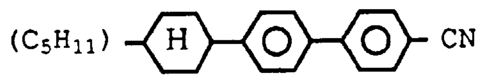

and pyrimidine type liquid crystal compounds such as

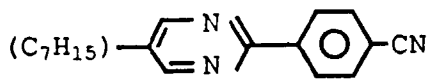

When a display element is formed by using the above-mentioned liquid crystal compounds, there can, for example, be employed additives which can be incorporated into ordinary liquid crystal compositions, for example, conductivity imparting agents and lifetime improving agents, in addition to the above-mentioned carboxylic acid ester compounds and other liquid crystal compounds.

The liquid crystal materials employable in the invention can be prepared using the above-mentioned carboxylic acid ester compounds. They can be also prepared by mixing the carboxylic acid ester compounds with other liquid crystal compounds and additives, if desired.

As shown in Fig. 5, the liquid crystal element of the invention filled with the above-mentioned liquid crystal material comprises basically a cell 33 which is composed of two transparent substrates 31a and 31b facing each other and so arranged as to form a gap 34 therebetween and transparent electrodes 32a and 32b each provided on the inner side of each transparent substrate; a liquid crystal material 35 filled in the gap 34; and polarizing plates 36a and 36b, each polarizing plate being arranged on each outer side of the cell 33.

Examples of transparent substrates are glasses and transparent polymer plates.

When using a glass substrate, an undercoat layer (i.e. a layer for preventing permeation of unnecessary components) containing, for example, silicon oxide as its host component may be provided on a surface of the glass substrate to inhibit deterioration of the liquid crystal material caused by eluation of alkali component from the glass substrate.

The transparent substrate, when it is a glass substrate, generally has a thickness of from 0.01 to 1.0 mm.

In the invention, flexible transparent substrates can be also employed as the transparent substrates. In this case, at least one of the transparent substrates may be a flexible transparent substrate or both of them may be flexible transparent substrates.

Examples of flexible transparent substrates are polymer films.

On the surface of each transparent substrate is provided a transparent electrode.

The transparent electrode can be formed by coating the surface of the transparent substrate with, for example, iridium oxide or tin oxide. The formation of the transparent electrode can be made utilizing conventionally known coating methods.

The thickness of the transparent electrode is generally 100 to 2,000 Å (10 to 200 nm).

On the transparent electrode which is provided on the transparent substrate as mentioned above may be provided an orientation controlling layer (i.e., orientation layer) or a ferroelectric layer.

The orientation layer can be formed, for example, by chemical adsorption of an organic silane coupling agent or a polynuclear complex of carboxylic acid, or by rhombic deposition of, for example, silicon oxide. The orientation layer can be also formed by applying a polyimide resin onto the transparent electrode, followed by rubbing the coated polyimide resin in a definite direction.

The orientation layer may be so formed as to serve simultaneously as a spacer as will be mentioned later.

Two sheets of the transparent substrate each having the transparent electrode thereon as illustrated above are

arranged in such a manner that the two sheets of the transparent electrodes face each other and form therebetween a gap which is to be filled with a liquid crystal material.

The width of the gap thus formed is usually 1 to 10 μm, preferably 1 to 5 μm. Such a gap as mentioned above can be easily formed, for example, by arranging two sheets of the substrates in such positions that a spacer is held therebetween.

As the spacer, there can be employed, for example, a polyimide type polymer material obtained by patterning a photosensitive polyimide precursor. By virtue of using such a spacer as mentioned above, a monodomain is formed by an interfacial effect between the spacer and the liquid crystal material. If a concentric circular or comb-like spacer which is serviceable as an orientation film is used, an orientation film and the spacer can be integrated into one system.

In order to form a gap between the two transparent substrates, a method of adding a fiber to the liquid crystal material can be also employed other than the method of using the above-mentioned spacer. In this method, a gap can be held between the substrates owing to the fiber.

In this case, moreover, the liquid crystal material can be mixed with particles instead of the fiber, or may be mixed with the fiber together with the particles.

The particles as referred to above include those made of melamine resin, urea resin or benzoguanamine resin having a particle diameter of from 1 to 10 μm.

The two sheets of the transparent substrate so arranged as to form a gap therebetween in the manner described above are then generally sealed on their peripheries using a sealing material. Examples of the sealing materials are epoxy resin and silicone resin, and they may be modified with, for example, an acrylic material or silicone rubber.

In the liquid crystal cell having the above-mentioned structure, the gap is filled with a liquid crystal material containing the aforementioned carboxylic acid ester compounds of formula [I], [II] or [III].

The liquid crystal material filled in the gap of the liquid crystal cell can be oriented utilizing, for example, a temperature gradient method using a spacer edge or a monoaxial orientation controlling method such as surface treatment using an orientation film. In the present invention, moreover, the initial orientation of the liquid crystal material can also be conducted by applying an electric field using a direct current bias voltage to the liquid crystal material while heating the liquid crystal material.

The liquid crystal cell filled with the liquid crystal material and initially oriented as mentioned above is placed between two polarizing plates. These two polarizing plates are arranged in such a manner that an angle formed by polarization planes of the polarizing plates would be 70-110°. Preferably, those two polarizing plates are so arranged that the polarization directions of the polarizing plates meet at a right angle, that is, the above-mentioned angle is 90°.

As the above-mentioned polarizing plates, employable are polarizing films prepared by stretching resin films such as a polyvinyl alcohol resin film and a polyvinyl butyral resin film in the presence of, for example, iodine to impart polarization to the stretched films. The polarizing films as illustrated above may be laminated on the surface with an other resin to have a multi-layer construction.

In the present invention, the liquid crystal cell can be placed between the two polarizing plates as arranged above in such a manner that the cell forms an angle (rotation angle) within the range of from +10° to -10° (hereinafter abbreviated to ±10°) from the state wherein the amount of transmitted light is the smallest (i.e. the darkest state), preferably in the darkest state. Alternatively, the liquid crystal cell can be placed in a state to form an angle (rotation angle) within the range of ±10° from the state wherein the amount of transmitted light is the largest (i.e. the brightest state), preferably in the brightest state.

Driving of the liquid crystal element of the present invention having a structure as mentioned above can be conducted by applying an electric field to the liquid crystal element.

In more detail, the liquid crystal element of the invention can be driven by applying thereto an electric field controlled to have a frequency of usually 1 Hz-100 kHz, preferably 10 Hz-10 kHz, and a voltage of usually 0.01-60 Vp-p/$\mu m^t$, preferably 0.05-30 Vp-p/$\mu m^t$ (voltage per 1 μm thickness).

When the liquid crystal element is driven by application of an electric field, the amount of light that transmits through this element exhibits two kinds of hysteresis curves by changing the wave form (driving wave) of the electric field applied thereto. That is, the present inventors have been successful in achieving memorization in one liquid crystal element by employing two kinds of driving methods. Of the two driving methods, one is to utilize a so-called double state stability, and the other is to utilize a so-called triple state stability.

A liquid crystal element using MHPOBC as a liquid crystal material is known as a liquid crystal element exhibiting triple state stability, but it exhibits practically no double state stability.

It has been realized first by the liquid crystal element of the present invention that either double state stability or triple state stability can be selected in one liquid crystal element only by the operation of changing the wave form (driving wave) of the electric field applied thereto.

Fig. 5 is an oscillograph of a wave form showing the relationship between the amount of transmitted light and the applied voltage in a liquid crystal element exhibiting triple state stability, and Fig. 6 is an oscillograph of a wave form showing the same relationship in a liquid crystal element exhibiting double state stability.

In the liquid crystal element used herein, a liquid crystal cell filled with a liquid crystal material is placed between two polarizing plates whose polarization planes meet at right angles, so that the darkest state of the element is attained without applying an electric field thereto. Fig. 5 shows an example of an oscillographic wave form obtained at the time when a triangular wave of 10 Hz is applied to this liquid crystal element, and Fig. 6 shows an example of an oscillographic wave form obtained at the time when a triangular wave of 100 Hz is applied thereto.

In the liquid crystal element wherein the liquid crystal cell and polarizing plates are arranged so as to attain the darkest state in the element as mentioned above, a favorable tri-stable state can be realized by application of an electric field of a relatively low frequency, for example, 0.001-50 Hz, preferably 0.1-30 Hz, to the element. The oscillographic wave form is gradually transformed into the bi-stable state as shown in Fig. 6 with increasing frequency of the electric field applied, and hence a favorable bi-stable state can be realized, for example, by applying an electric field having a frequency of 50 Hz-100 kHz, preferably 70 Hz-10 kHz to the liquid crystal element.

In the liquid crystal element as mentioned above, for example, as shown in Fig. 5, a dark state can be attained when the applied voltage is 0 Vp-p, and in this case the contrast becomes markedly high.

Figs. 7 and 8 each show an oscillographic wave form of a liquid crystal element in which a liquid crystal cell filled with a liquid crystal material is placed between two polarizing plates whose polarization planes meet at a right angle so that the brightest state of the element is attained. Fig. 7 shows an example of an oscillographic wave form obtained at the time when a triangular wave of 10 Hz is applied to the liquid crystal element, and Fig. 8 shows an example of an oscillographic wave form obtained at the time when a triangular wave of 100 Hz is applied to the liquid crystal element. In this liquid crystal element, there is a tendency similar to that in the above-mentioned liquid crystal elements, for example, a bi-stable state is attained by applying an electric field having a relatively high frequency.

The electric field applied to the above-mentioned liquid crystal elements is preferably selected from among a rectangular wave (or pulse wave), triangular wave, sinusoidal wave (sine wave) and a combination thereof. When a rectangular wave (or pulse wave or a combination of both) is applied to the liquid crystal element, the speed for driving the liquid crystal element can be increased by reducing the width of the applied electric field to not more than 10 msec, preferably to from 0.01 to 10 msec. In this region, the liquid crystal element of the invention may be used as a bi-stable state type liquid crystal element. Further, by employing this electric field having a width of larger than 10 msec, preferably of from 33 to 1,000 msec, the liquid crystal element of the invention can be used as a tri-stable state type liquid crystal element in the region where high driving is not required. The width of an electric field used herein is intended to designate, for example in a rectangular wave, a length (i.e. time) of the electric field maintained at a given voltage.

To this liquid crystal element, an electric field can be applied while varying the voltage between a negative voltage and a positive voltage through OV. In the driving method for developing such a bi-stable state as shown in Figs. 6 and 8, a hysteresis curve showing a favorable double state stability can be formed, for example, by varying the applied voltage between -30 V and +30 V. In the liquid crystal element showing a triple state stability, an electric field can be applied in the manner similar to that of the above-mentioned case.

Further, to this liquid crystal element may be applied an electric field having the above-mentioned wave form by varying the voltage between 0 and a positive voltage, or by varying the voltage between 0 and a negative voltage. Namely, there can be employed a light modulation method utilizing, for example, the light transmission properties shown by a hysteresis curve formed on a plus voltage side by application of an electric field wherein the voltage is varied in the range of 0 to +30 V. Similarly, there can be also employed a light modulation method utilizing light transmission properties shown by a hysteresis curve formed at a minus voltage side by application of an electric field wherein the voltage is varied in the range of 0 to -30 V.

The liquid crystal element of the invention is superior to liquid crystal elements of the prior art in that the element can be driven by utilizing two kinds of driving methods as mentioned above, and the memory effect of the element can be assured by suitably selecting a desired driving method out of the two methods according to the conditions under which the element is driven.

The liquid crystal element as mentioned above can be used as an ordinary liquid crystal element, but it is particularly useful as a display element.

Examples of the display elements include a large frame display, a multi-information display for automobiles, and a navigation display for automobiles. These display elements can be driven, according to the purposes for which they are used, by the above-mentioned driving methods as a bi-stable state type liquid crystal element or a tri-stable state type liquid crystal element.

According to the liquid crystal elements of the invention and the light modulation methods using the liquid crystal elements, stable states of two forms, namely a bi-stable state and a tri-stable state, can be attained by using only one kind of liquid crystal element.

Further, when the liquid crystal elements of the invention are used, a dark state having a sufficient darkness can be attained, and hence not only the contrast between the bright state and dark state can be prominently enhanced, but also a favorable memory effect can be assured.

The carboxylic acid ester compounds of formulae [I], [II] and [III] employable in the liquid crystal elements are

optically active. Moreover, the carboxylic acid ester compounds of formulae [I] and [II] have specific structure in which a ring structure such as a naphthalene ring structure is combined, and this specific structure is rigid. Hence, these compounds have smectic phase over a wide temperature range in the vicinity of room temperature, and can favorably be used as anti-ferroelectric liquid crystal compounds.

By virtue of mixing the liquid crystal compounds with the same kinds of liquid crystal compounds and/or different kinds of liquid crystal compounds, the temperature for actuation of the liquid crystal phase of the liquid crystal compounds can be reduced or the temperature range therefor can be broadened without marring the anti-ferroelectric properties of the liquid crystal compounds.

Accordingly, the liquid crystal elements having a high speed response over a wide temperature range can be obtained by using the above-mentioned liquid crystal compounds or the liquid crystal compositions containing the liquid crystal compounds.

The liquid crystal compounds or the liquid crystal compositions containing the liquid crystal compounds can be favorably employed over a wide temperature range in the vicinity of room temperature as ferroelectric liquid crystal compounds or compositions.

Further, in the case of producing liquid crystal display devices using the above-mentioned liquid crystal elements, the time for scanning the display devices is markedly shortened.

Furthermore, the liquid crystal compounds of the invention have spontaneous polarization, and hence there can be obtained liquid crystal elements having a favorable memory effect even after elimination of an electric field by using a thin film cell filled with the liquid crystal compounds.

When such liquid crystal elements are used, power consumption can be reduced, a stable contrast can be obtained, and also low voltage driving can be performed. The liquid crystal elements utilize the smectic phase of the carboxylic acid ester compounds, so that they can be favorably employed for optical switching elements which are generally used over a wide temperature range.

The present invention is now further described in the following Examples. The symbols "R" and "S" used in the Examples mean R modification and S modification, respectively.

Example 1

Synthesis of the 1'''-trifluoromethylheptyl ester of trans-4-[4"-(6"-n-decyloxy-2"-naphthoyloxy)phenyl]cyclocarboxylic acid

First step

A mixture of 2.20 g (10 mmol) of trans-4-(4'-hydroxyphenyl)cyclohexane carboxylic acid, 6.84 g (40 mmol) of benzyl bromide, 5.53 g (40 mmol) of potassium carbonate and 50 ml of dimethylformamide was stirred for 4 hours at 120 °C, and then stirred for another 4 hours at room temperature.

The obtained reaction mixture was added to 450 ml of water, and the resulting mixture was filtered to obtain a viscous liquid. The viscous liquid was washed with hexane to obtain 2.54 g (6.4 mmol) of the benzyl ester of trans-4-(4'-benzoyloxyphenyl)cyclohexane carboxylic acid.

Second step

A mixture of 2.54 g (6.4 mmol) of the benzyl ester of trans-4-(4'-benzoyloxyphenyl)cyclohexane carboxylic acid obtained in the first step, 0.86 g (13 mmol) of 85 % aqueous solution of potassium hydroxide, 30 ml of ethanol and 30 ml of water was stirred for 9 hours at 120 °C.

To the reaction mixture was added 200 ml of water and then hydrochloric acid was added dropwise to acidify the system, so as to obtain a white solid. The obtained white solid was filtered to obtain 1.68 g (5.4 mmol) of trans-4-(4'-benzyloxyphenyl)cyclohexane carboxylic acid in the form of a white solid.

Third step

To a mixture of 1.68 g (5.4 mmol) of trans-4-(4'-benzyloxyphenyl)cyclohexane carboxylic acid obtained in the second step, 0.994 g (5.4 mmol) of R-1-trifluoromethylheptyl alcohol, 0.12 g (1 mmol) of 4-N,N-dimethylaminopyridine and 20 ml of methylene chloride there was added dropwise 10.8 ml of methylene chloride solution containing 1.11 g (5.4 mmol) of N,N'-dicyclohexylcarbodiimide over 1 hour while stirring the mixture.

The reaction was carried out at room temperature for an additional 10 hours.

The reaction mixture was filtered, and the obtained filtrate was concentrated. The concentrate was separated using column chromatography to obtain 2.27 g (4.76 mmol) of the R-1"-trifluoromethylheptyl ester of trans-4-(4'-benzyloxyphenyl)cyclohexane carboxylic acid in the form of a white solid.

Fourth step

Hydrogen gas was passed through a mixture of 2.27 g (4.76 mmol) of the R-1"-trifluoromethylheptyl ester of trans-4-(4'-benzyloxyphenyl)cyclohexane carboxylic acid obtained in the third step, 1.6 g of a 5 % palladium-carbon catalyst and 57 ml of tetrahydrofuran with stirring at room temperature for 6.5 hours.

Subsequently, the reaction mixture was filtered using Celite, which is a filtration assistant, and the obtained filtrate was concentrated. The concentrate was separated using column chromatography to obtain 1.90 g (4.76 mmol) of the R-1"-trifluoromethylheptyl ester of trans-4-(4'-hydroxyphenyl)cyclohexane carboxylic acid in the form of a colorless viscous liquid.

Fifth step

To a mixture of 0.386 g (1 mmol) of the R-1"-trifluoromethylheptyl ester of trans-4-(4'-hydroxyphenyl)cyclohexane carboxylic acid obtained in the fourth step, 0.328 g (1 mmol) of 6-n-decyloxynaphthalene-2-carboxylic acid, 0.012 g (0.1 mmol) of 4-N,N-dimethylaminopyridine and 10 ml of methylene chloride there was added dropwise 2 ml of methylene chloride solution containing 0.21 g (0.1 mmol) of N,N'-dicyclohexylcarbodiimide with stirring at room temperature over a period of one hour.

The mixture was allowed to undergo reaction at room temperature for 8 hours.

The reaction mixture was filtered, and the obtained filtrate was concentrated. The concentrate was separated using column chromatography to obtain 0.58 g of a white solid.

FD-mass spectrum of this white solid was measured to obtain an M/e value of 696.

Fig. 3 shows a chart of the $^1$H-NMR spectrum of this compound.

From the results of the analyses, the compound was identified as the R-1"'-trifluoromethylheptyl ester of trans-4-[4'-(6"-n-decyloxy-2"-naphthoyloxy)phenyl]cyclohexyl carboxylic acid which was the desired compound, i.e. exemplified compound (115).

Example 2

Synthesis of the R-1"'-trifluoromethylheptyl ester of 4'-(6"-decyloxy-2"-naphthoyloxy)biphenyl-4-carboxylic acid

First step

A mixture of 6.42 g (30 mmol) of 4'-benzoxybiphenyl-4-carboxylic acid, 20.52 g (120 mmol) of benzyl bromide, 16.58 g (120 mmol) of potassium carbonate and 40 ml of dimethylformamide was stirred for 2.5 hours at 120 °C, and then further stirred for 4.5 hours at room temperature.

The obtained reaction mixture was added to 500 ml of water, and the resulting mixture was subjected to filtration to obtain the benzyl ester of 4'-benzyloxybiphenyl-4-carboxylic acid in the form of a white solid.

Second step

A mixture of 11.82 g (30 mmol) of the benzyl ester of 4'-benzyloxybiphenyl-4-carboxylic acid obtained in the first step, 3.96 g (60 mmol) of 85 % aqueous solution of potassium hydroxide, 30 ml of ethanol and 30 ml of water was heated under reflux for 4 hours.

The reaction mixture was added to 400 ml of water, and then to the mixture there was added hydrochloric acid to acidify the system, so as to obtain a white solid. Then, the white solid was filtered to obtain 7.95 g (26.2 mmol) of 4'-benzyloxybiphenyl-4-carboxylic acid in the form of a white solid.

Third step

To a mixture of 1.50 g (5 mmol) of 4'-benzyloxybiphenyl-4-carboxylic acid obtained in the second step, 0.92 g (5 mmol) of R-1-trifluoromethylheptyl alcohol, 0.122 g (1 mmol) of 4-N,N-dimethylaminopyridine and 30 ml of methylene chloride there was added dropwise 10 ml of methylene chloride solution containing 1.03 g (5 mmol) of N,N'-dicyclohexylcarbodiimide over 3 hours while stirring the mixture.

The reaction was carried out at room temperature for an additional 4 hours.

The reaction mixture was filtered, and the obtained filtrate was concentrated. The concentrate was separated using column chromatography to obtain 1.17 g (2.5 mmol) of the R-1"-trifluoromethylheptyl ester of 4'-benzyloxybiphenyl-4-carboxylic acid.

Fourth step

Hydrogen gas was passed through a mixture of 1.15 g (2.44 mmol) of the R-1"-trifluoromethylheptyl ester of 4'-benzyloxybiphenyl-4-carboxylic acid obtained in the third step, 0.9 g of a 5 % palladium-carbon catalyst and 50 ml of tetrahydrofuran with stirring at room temperature for 6 hours.

Subsequently, the reaction mixture was filtered using Celite, which is a filtration assistant, and the obtained filtrate was concentrated. The concentrate was separated using column chromatography to obtain 0.92 g (2.44 mmol) of the R-1"-trifluoromethylheptyl ester of 4'-hydroxybiphenyl-4-carboxylic acid in the form of a colorless liquid.

Fifth step

To a mixture of 0.38 g (1 mmol) of the R-1"-trifluoromethylheptyl ester of 4'-hydroxybiphenyl-4-carboxylic acid obtained in the fourth step, 0.328 g (1 mmol) of 6-n-decyloxynaphthlene-2-carboxylic acid, 0.012 g (0.1 mmol) of 4-N, N-dimethylaminopyridine and 20 ml of methylene chloride there was added dropwise 2 ml of methylene chloride solution containing 0.21 g (0.1 mmol) of N,N'-dicyclohexylcarbodiimide with stirring at room temperature over a period of one hour.

The mixture was allowed to undergo reaction at room temperature for 8 hours.

The reaction mixture was filtered, and the obtained filtrate was concentrated. The concentrate was separated using column chromatography to obtain 0.03 g of a white solid.

The FD-mass spectrum of this white solid was measured to obtain an M/e value of 690.

From the results of the analysis, the compound was identified as the R-1"'-trifluoromethylheptyl ester of 4'-(6"-decyloxy-2"-naphthoyloxy)-biphenyl-4-carboxylic acid i.e., exemplified compound [139].

Example 3

Synthesis of the R-1"'-methylheptyl ester of 4-[4'-(6"-decyloxy-2"-naphthoyloxy)benzoyloxy]-benzoic acid First step

To a mixture of 1.62 g (5 mmol) of 6-decyloxynaphthalene-2-carboxylic acid, 1.14 g (5 mmol) of the benzyl ester of 4-hydroxybenzoic acid, 0.12 g (1 mmol) of 4-N,N-dimethylaminopyridine and 20 ml of methylene chloride there was added dropwise 10 ml of methylene chloride solution containing 1.03 g (5 mmol) of N,N'-dicyclohexylcarbodiimide over 1 hour while stirring the mixture.

The reaction was carried out at room temperature for an additional 10 hours.

The reaction mixture was filtered, and the obtained filtrate was concentrated. The concentrate was separated using column chromatography to obtain 2.30 g (4.28 mmol) of the benzyl ester of 4-(6'-n-decyloxy-2'-naphthoyloxy)benzoic acid in the form of a white solid.

Second step

Hydrogen gas was passed through a mixture of 2.15 g (4 mmol) of the benzyl ester of 4-(6'-n-decyloxy-2'-naphthoyloxy)benzoic acid obtained in the first step, 1 g of a 5 % palladium-carbon catalyst and 30 ml of tetrahydrofuran with stirring at room temperature for 8 hours.

Subsequently, the reaction mixture was filtered using Celite, which is a filtration assistant, and the obtained filtrate was concentrated to obtain 1.57 g (3.52 mmol) of 4-(6'-n-decyloxy-2'-naphthoyloxy)benzoic acid.

Third step

To a mixture of 0.45 g (1 mmol) of 4-(6'-n-decyloxy-2'-naphthoyloxy)benzoic acid obtained in the second step, 0.21 g (1 mmol) of the R-1'-methylheptyl ester of 4-hydroxybenzoic acid, 0.012 g (0.1 mmol) of 4-N,N-dimethylaminopyridine and 10 ml of methylene chloride there was added dropwise 2 ml of methylene chloride solution containing 0.21 g (0.1 mmol) of N,N'-dicyclohexylcarbodiimide with stirring at room temperature over a period of one hour.

The mixture was allowed to undergo reaction at room temperature for another 8 hours.

The reaction mixture was filtered, and the obtained filtrate was concentrated. The concentrate was separated using column chromatography to obtain 0.52 g of a colourless semi-solid.

The FD-mass spectrum of this semi-solid was measured to obtain an M/e value of 696.

From the results of the analyses, the compound was identified as the R-1"'-methylheptyl ester of 4-[4'-(6"-n-decyloxy-2"-naphthoyloxy)benzoyloxy]-benzoic acid, i.e., exemplified compound [205].

Example 4

The phase transition temperatures of the compound [205] obtained in Example 3 were measured. The results are set forth in the aforementioned Table 3.

Example 5

A composition composed of 49 wt.% of the compound obtained in Example 3 [205] and 51 wt.% of a compound having the following formula [304] was prepared, and the phase transition temperatures of the composition were measured.

The results are set forth in Table 4.

Table 4

| Compound or Composition | Phase Trasition Temperature (°C) | | | | | | |
|---|---|---|---|---|---|---|---|
| | Cry | | SmC* | | SmA | | Iso |
| [205] + [304] 49wt% 51wt% | • | 57 | • | 127 | • | 184 | • |
| [205] | • | 100 | • | 137 | • | 181 | • |
| [304] | • | 73 | • | 125 | • | 183 | • |

The compound [304] has the following formula:

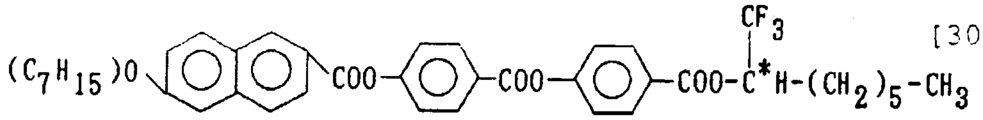

[304]

Example 6

Synthesis of the R-1'''-trifluoromethylheptyl ester of 4-[4'-(6"-n-heptyloxy-2"-naphthoyloxy)benzoyloxy]benzoic acid

First step

To a mixture of 1.4 g (5 mmol) of 4-benzyloxybenzoic acid, 0.92 g (5 mmol) of R-1-trifluoromethylheptanol, 0.12 g (1 mmol) of 4-N,N-dimethylaminopyridine and 20 ml of methylene chloride there was added dropwise 10 ml of methylene chloride solution containing 1.03 g (5 mmol) of N,N'-dicyclohexylcarbodiimide over 1 hour under stirring of the mixture.

The reaction was continued at room temperature for an additional 10 hours.

The reaction mixture was filtered and the obtained filtrate was concentrated. Using column chromatography, the concentrate was separated to obtain 1.75 g (4.4 mmol) of the R-1'-trifluoromethylheptyl ester of 4-benzyloxybenzoic acid.

Second step

Hydrogen gas was passed through a mixture of 1.58 g (4 mmol) of the R-1'-trifluoromethylheptyl ester of 4-benzyloxybenzoic acid obtained in the first step, 0.5 g of a 5 % palladium-carbon and 30 ml of ethyl acetate with stirring at room temperature under normal pressure for 9 hours. The reaction mixture was filtered using Celite, which is a filtration assistant, and the obtained filtrate was concentrated.

Using column chromatography, the concentrate was separated to obtain 1.75 g (4.4 mmol) of the R-1'-trifluoromethylheptyl ester of 4-hydroxybenzoic acid in the form of a colourless viscous liquid.

Third step

To a mixture of 2.43 g (8.5 mmol) of 6-n-heptyloxynaphthalene-2-carboxylic acid, 0.012 g (0.1 mmol) of 4-N,N-dimethylaminopyridine and 30 ml of methylene chloride there was added dropwise 10 ml of methylene chloride solution containing 1.75 g (8.5 mmol) of N,N'-dicyclohexylcarbodiimide over 1 hour while stirring and cooling the mixture with ice.

The reaction mixture was filtered, and the obtained filtrate was concentrated. Using column chromatography, the concentrate was separated to obtain 2.72 g (5.5 mmol) of the benzyl ester of 4-(6'-heptyloxy-2'-naphthoyloxy)benzoic acid.

Fourth step

Hydrogen gas was passed through a mixture of 2.33 g (4.7 mmol) of the benzyl ester of 4-(6'-heptyloxy-2'-naphthoyloxy)benzoic acid obtained in the third step, 1.0 g of a 5 % palladium-carbon and 30 ml of tetrahydrofuran with stirring at room temperature under normal pressure for 8 hours.

The reaction mixture was filtered using Celite, which is a filtration assistant, and the obtained filtrate was concentrated. The concentrate was then recrystallized using toluene to obtain 1.42 g (3.5 mmol) of 4-(6'-n-heptyloxy-2-naphthoyloxy)benzoic acid in the form of a white solid.

Fifth step

To a mixture of 0.41 g (1 mmol) of 4-(6'-n-heptyloxy-2-naphthoyloxy)benzoic acid obtained in the fourth step, 0.30 g (1 mmol) of the R-1'-trifluoromethylheptyl ester of 4-hydroxybenzoic acid, 0.012 g (0.1 mmol) of 4-N,N-dimethylaminopyridine and 10 ml of methylene chloride there was added dropwise 2 ml of methylene chloride solution containing 0.21 g (0.1 mmol) of N,N'-dicyclohexylcarbodiimide with stirring at room temperature over a period of one hour.

The mixture was allowed to undergo reaction at room temperature for 8 hours.

The reaction mixture was filtered, and the obtained filtrate was concentrated. The concentrate was separated using column chromatography to obtain 0.63 g of a colourless semi-solid.

The FD-mass spectrum of this semi-solid was measured to obtain an M/e value of 739.

From this result and the analysis of the $^1$H-NMR spectrum of this compound, this compound was identified as the R-1'''-trifluoromethylheptyl ester of 4-[4'-(6''-n-heptyloxy-2''-naphthoyloxy)benzoyloxy]benzoic acid, which was the desired compound, i.e., exemplified compound [304].

The carboxylic acid ester compound of formula [304] prepared as above and the compound represented by the following formula (Cr-1) were mixed with each other in a ratio of 51 : 49, by weight, to prepare a liquid crystal material (i.e., a liquid crystal composition).

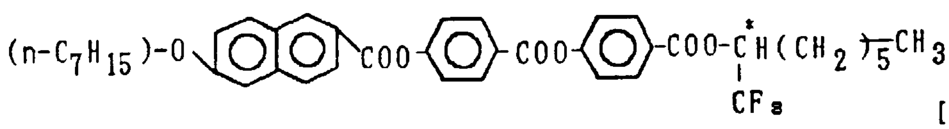

[304]

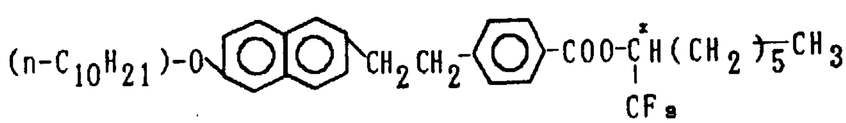

(Cr-1)

The phase transition temperatures of this liquid crystal composition are set forth in Table 5. In the Table the phase transition temperatures of the compound of formula (Cr-1) are also set forth.

Table 5

| Liquid Crystal Material | Phase Trasition Temperature (°C) | | | | | | |
|---|---|---|---|---|---|---|---|
| | Cry | | SmC* | | SmA | | Iso |
| Formula [304] | • | 73 | • | 125 | • | 183 | • |
| Composition | • | <-30 | • | 52 | • | 96 | • |
| (Cr-1) | • | -13 | • | | • | -6 | • |

Example 7

The carboxylic acid ester compound of formula [302] was melted and introduced into a gap (kept under reduced pressure) of a cell, the cell being composed of two substrates with ITO transparent electrodes, each substrate being provided with an orientation controlling film (thickness: 150 Å (15 nm)) made of polyimide (PIQ-5400, a product of Hitachi Kasei Kogyo K.K.) on the inner surface thereof as shown in Fig. 4. The two polyimide films were so rubbed as to have orientation directions thereof nearly parallel to each other and in the same direction. The cell thus filled with the liquid crystal material was heated to 200 °C, kept at 200 °C for 5 minutes, and then cooled at a rate of 1 °C/min to 60 °C, to prepare a liquid crystal element.

The Cell conditions are as follows:

(a) External size: 2.5 cm long X 2.2 cm width X 1.5 mm thick
(b) Substrate: 0.7 mm thick, material: glass
(c) Distance between substrates: 2 μm
(d) Sidewall size: 1.8 mm long X 0.1 cm width X 2 μm thick

The above-mentioned cell was prepared in the following manner.

Polyimide was applied to a glass substrate having an ITO transparent electrode film thereon. Coating of the polyimide (PIQ-5400, a product of Hitachi Kasei Kogyo K.K.) on the ITO transparent electrode was conducted by a spin coating method. In detail, the polyimide was diluted with N-methylpyrrolidone to prepare a 1.2 % solution, and the solution was then spin-coated at 2000 r.p.m. The polyimide solution thus coated was cured by heating at 325 °C for 30 minutes, whereupon a polyimide film of 150 to 200 Å (15 to 20 nm)) thickness was formed. The polyimide film was then rubbed with a nylon cloth in one direction, thereby imparting an ability of orientating the liquid crystal thereto.

Two sheets of the polyimide film-coated glass substrate prepared as above were put on each other to prepare a cell. That is, an epoxy adhesive was applied to each of the polyimide film-coated glass substrates by silk screen printing in order to bond the two substrates to each other and to control a gap of the cell. The epoxy adhesive was prepared by mixing an adhesive base (LCB-304B, a product of EHC) with a curing agent (LCB-304B, a product of EHC) and beads (GP-20, a product of EHC) for controlling the cell gap in a proportion of 130 : 30 : 3.

One of the glass substrates was coated with the epoxy adhesive and laminated to other glass substrate in such a manner that the polyimide films faced each other. The epoxy adhesive thus coated was cured under such curing conditions that heating was conducted at 50 °C for 15 minutes, at 60 °C for 15 minutes, at 70 °C for 15 minutes, at 80 °C for 15 minutes, at 125 °C for 30 minutes and at 170 °C for 60 minutes, to combine the substrates with each other.

The thus prepared cell having a gap of about 2 μm was filled with the liquid crystal material prepared as above, and the cell filled with the liquid crystal material was evaluated.

A liquid crystal element was prepared by placing the above-mentioned liquid crystal cell filled with the liquid crystal material between two polarizing plates whose polarization planes meet at right angles so that the darkest state was attained in the element.

A triangular wave of 30 $V_{p-p}$ was applied to the liquid crystal element to measure the intensity of transmitted light, and as a result, an oscillographic wave shown in Fig. 5 was obtained by application of a frequency of 10 Hz and, an oscillographic wave shown in Fig. 6 was obtained by application of a frequency of 100 Hz.

As is clear from Fig. 5, this liquid crystal element attained a contrast of 34 between the time when 0 V was applied and the time when +30 V (or -30 V) was applied by application of a triangular wave of 10 Hz.

As is clear from Fig. 6, this liquid crystal element attained a contrast of 15 between the time when -12 V was applied and the time when +12 V was applied by application a triangular wave of 100 Hz.

In the liquid crystal element of the invention in which the liquid crystal cell is placed between two polarizing plates whose polarization planes meet at right angles in such a manner that the darkest state is attained in the element, the dark state can be attained by applying a voltage of 0 V using a low frequency, in particular, whereby the contrast between the dark state and the bright state can be prominently enhanced.

The contrast mentioned above was determined by measuring the intensity of the transmitted light in the bright state and in the dark state while changing the voltage applied to the liquid crystal element, and calculating therefrom the proportion of I (bright state) / I (dark state).

Subsequently, a liquid crystal element was prepared by placing the above-mentioned liquid crystal cell filled with the liquid crystal material between two polarizing plates whose polarization planes meet at right angles in such a manner that the brightest state was attained in the element.

A triangular wave of 30 $V_{p-p}$ was applied to the liquid crystal element to measure the intensity of transmitted light, and as a result, an oscillographic wave shown in Fig. 7 was obtained by application of a frequency of 10 Hz, and an oscillographic wave shown in Fig. 8 was obtained by application of a frequency of 100 Hz.

From the results, it was confirmed that the liquid crystal element of the invention in which the liquid crystal cell is

placed between two polarizing plates whose polarization planes meet at right angles in such a manner that the brightest state is attained in the element was able to secure a favorable memory effect using a high frequency, in particular.

## Claims

1. A carboxylic acid ester compound represented by formula [I]:

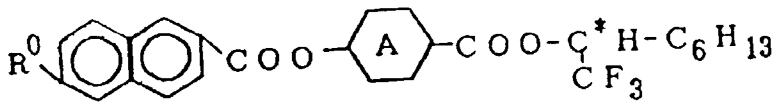

[I]

wherein $R^0$ is an alkyl group of 3-20 carbon atoms, alkoxy group of 3-20 carbon atoms or halogenated alkyl group of 3-20 carbon atoms; C* is an asymmetric carbon atom; and

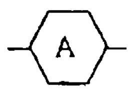

represents a group selected from

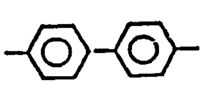 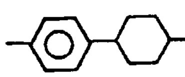 and 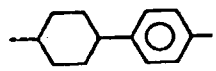

2. A carboxylic acid ester compound represented by formula [II]:

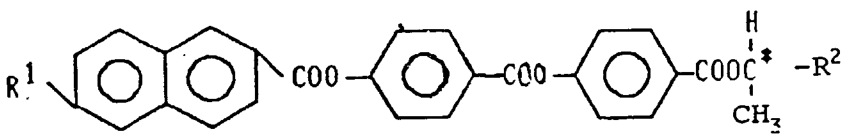

[II]

wherein $R^1$ is an alkyl group of 3-20 carbon atoms, alkoxy group of 3-20 carbon atoms or halogenated alkyl group of 3-20 carbon atoms; C* is an asymmetric carbon atom; and $R^2$ is an alkyl group of 2-10 carbon atoms.

3. Use of a compound of formula [I] or [II] as defined in claim 1 or 2 as a liquid crystal compound.

4. A liquid crystal composition comprising a compound of formula [I] or [II] as defined in claim 1 or 2.

5. A liquid crystal element comprising a cell and two polarizing plates, one on each outer side of the cell, said cell being composed of two transparent substrates so arranged as to face each other leaving a gap therebetween and a transparent electrode provided on the inner surface of each substrate, said gap being filled with a liquid crystal material, wherein:

said two polarizing plates are so arranged that an angle formed by polarization planes of the polarizing plates is in the range of 70°-110°, and said cell filled with the liquid crystal material is arranged between the polarizing plates at an angle of from +10° to -10° relative to the position of the cell at which the transmitted light becomes the darkest or the brightest;
and
said liquid crystal material comprising a liquid crystal compound represented by formula [III]:

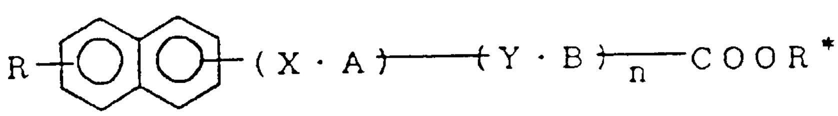

[III]

wherein R is an alkyl group of 3-20 carbon atoms, alkoxy group of 3-20 carbon atoms or halogenated alkyl group of 3-20 carbon atoms; X and Y each represent a group selected from -COO-, -OCO-, $-CH_2CH_2-$, $-CH_2O-$, $-OCH_2-$,

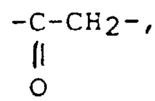, 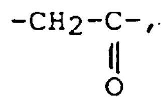,

and -S-S-, or a single bond;
R* represents a group selected from

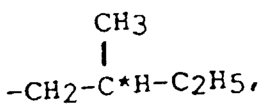, 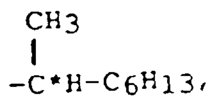, 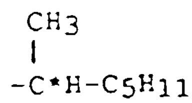, 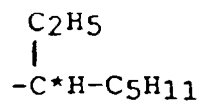,

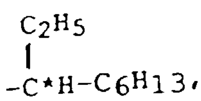, 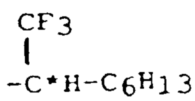 and 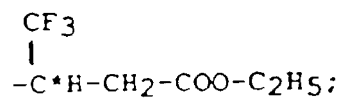;

n is an integer of 1 or 0; and when n=0, A is

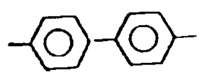 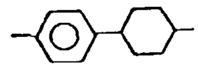 or 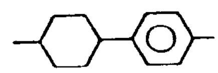

and when n=1, A and B each is

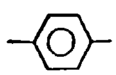, 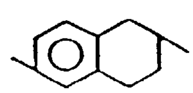, 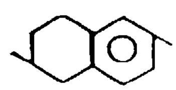 or 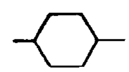;

6. An element according to claim 5 wherein the compound of formula [III] is a compound as claimed in claim 1 or 2.

7. A light modulation method which comprises applying an electric field to a liquid crystal element as claimed in claim 5 or 6.

8. A method according to claim 7, wherein the amount of a light transmitted by the liquid crystal element is changed by varying the voltage applied to the element between a positive voltage and a negative voltage.

9. A method according to claim 7 wherein the amount of a light transmitted by the liquid crystal element is changed by varying the voltage applied to the liquid crystal element between 0 and a positive voltage, or between 0 and a negative voltage.

10. A method according to claim 8 or 9 wherein the electric field is of rectangular wave form, triangular wave form, sine wave form or a combination of such wave forms.

11. A method according to claim 10 wherein the electric field is of rectangular wave form in which minimum width of the rectangular wave is not more than 10 msec.

12. A display element using a liquid crystal element as claimed in claim 5 or 6.

13. A display method utilizing a light modulation method such as claimed in any one of claims 7 to 11.

**Patentansprüche**

1. Carbonsäureesterverbindung, angegeben durch die Formel [I]:

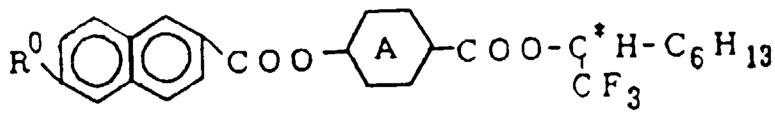

[I]

in der $R^0$ eine Alkylgruppe mit 3 bis 20 Kohlenstoffatomen, Alkoxygruppe mit 3 bis 20 Kohlenstoffatomen oder halogenierte Alkylgruppe mit 3 bis 20 Kohlenstoffatomen bedeutet; C* ein asymetrisches Kohlenstoffatom ist, und

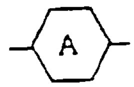

eine Gruppe bedeutet, ausgewählt aus

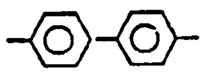 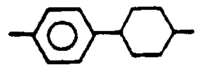 und 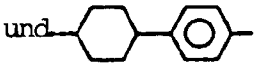

**2.** Carbonsäureesterverbindung, angegeben durch die Formel [II] :

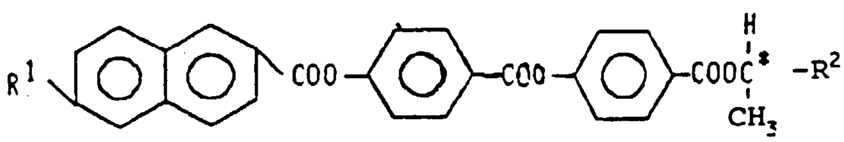

(II)

wobei $R^1$ eine Alkylgruppe mit 3 bis 20 Kohlenstoffatomen, Alkoxygruppe mit 3 bis 20 Kohlenstoffatomen oder halogenierte Alkylgruppe mit 3 bis 20 Kohlenstoffatomen bedeutet; C* ein asymetrisches Kohlenstoffatom ist und $R^2$ eine Alkylgruppe mit 2 bis 10 Kohlenstoffatomen.

**3.** Verwendung einer Verbindung der Formel [I] oder [II], wie in Anspruch 1 oder 2 definiert, als Flüssigkristall-Verbindung.

**4.** Flüssigkristall-Masse, umfassend eine Verbindung der Formel [I] oder [II], wie in Anspruch 1 oder 2 definiert.

**5.** Flüssigkristall-Element, umfassend eine Zelle und zwei polarisierende Platten, eine auf jeder Außenseite der Zelle, wobei die Zelle aus zwei transparenten Substraten besteht, die so angeordnet sind, daß sie einander gegenüberliegen und einen Zwischenraum dazwischen bilden, und einertransparentenElektrode, die auf der Innenseite jedes Substrates angeordnet ist, wobei der Zwischenraum mit einem Flüssigkristall-Material ausgefüllt ist, wobei

die beiden polarisierenden Platten so angeordnet sind, daß ein durch die Polarisationsebenen der polarisierenden Platten gebildeter Winkel im Bereich von 70° bis 110° liegt, und die mit dem Flüssigkristall-Material gefüllte Zelle zwischen den polarisierenden Platten in einem Winkel von +10° bis -10°, bezogen auf die Stellung der Zelle, in der das durchgehende Licht am dunkelsten oder am hellsten wird, angeordnet ist, und

das Flüssigkristall-Material eine Flüssigkristall-Verbindung umfaßt, angegeben durch die Formel [III]:

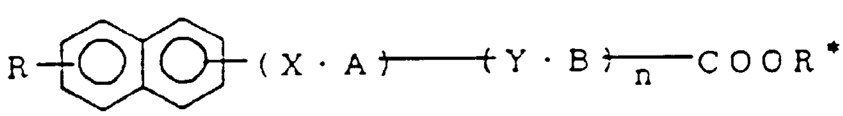

[III]

wobei R eine Alkylgruppe mit 3 bis 20 Kohlenstoffatomen, Alkoxygruppe mit 3 bis 20 Kohlenstoffatomen oder halogenierte Alkylgruppe mit 3 bis 20 Kohlenstoffatomen ist; X und Y jeweils eine Gruppe, ausgewählt aus -COO-, -OCO-, $-CH_2CH_2-$, $-CH_2O-$, $-OCH_2-$,

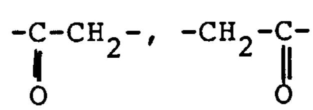

und -S-S- oder eine Einzelbindung bedeuten;

$R^*$ eine Gruppe bedeutet, ausgewählt aus

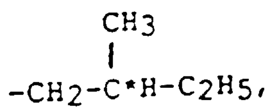 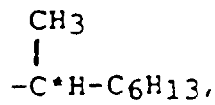 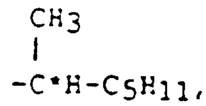 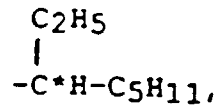

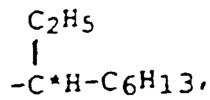 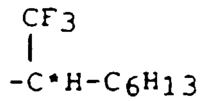 und 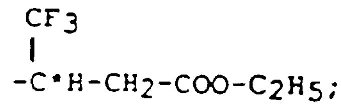

n eine ganze Zahl von 1 oder 0 ist, und wenn 0 ist, A

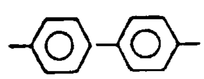 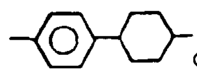 oder 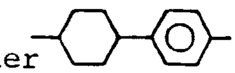

ist, und wenn n = 1 ist, A und B jeweils

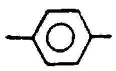 , 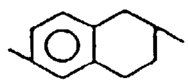 , 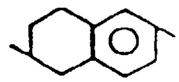 oder 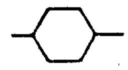 ;

ist.

6. Element nach Anspruch 5, wobei die Verbindung der Formel [III] eine Verbindung nach Anspruch 1 oder 2 ist.

7. Methode zur Lichtmodulation, umfassend das Anlegen eines elektrischen Feldes an ein Flüssigkristall-Element nach Anspruch 5 oder 6.

8. Methode nach Anspruch 7, wobei die Menge des durch das Flüssigkristall-Element durchgelassenen Lichtes verändert wird durch Variation der an das Element angelegten Spannung zwischen einer positiven Spannung und einer negativen Spannung.

9. Methode nach Anspruch 7, wobei die Menge des durch das Flüssigkristall-Element hindurchgehenden Lichtes verändert wird durch Veränderung der an das Flüssigkristall-Element angelegten Spannung zwischen 0 und einer positiven Spannung oder zwischen 0 und einer negativen Spannung.

10. Methode nach Anspruch 8 oder 9, wobei das elektrische Feld eine rechteckige Wellenform, dreieckige Wellenform, sinusförmige Wellenform oder eine Kombination derartiger Wellenformen besitzt.

11. Methode nach Anspruch 10, wobei das elektrische Feld eine rechteckige Wellenform besitzt, bei der die minimale Breite der rechteckigen Welle nicht mehr als 10 mSek. beträgt.

12. Displayelement unter Verwendung eines Flüssigkristall-Elementes nach Anspruch 5 oder 6.

13. Displaymethode unter Anwendung einer Lichtmodulationsmethode nach einem der Ansprüche 7 bis 11.

**Revendications**

1. Composé d'ester d'acide carboxylique représenté par la formule [I] :

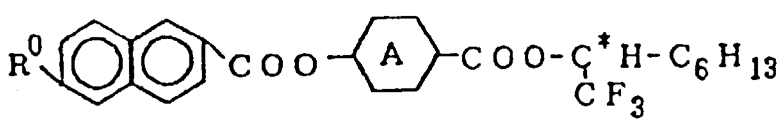 [I]

dans laquelle $R^0$ représente un groupe alkyle comportant de 3 à 20 atomes de carbone, un groupe alcoxy comportant de 3 à 20 atomes de carbone ou un groupe alkyle halogéné comportant de 3 à 20 atomes de carbone ; C* représente un atome de carbone asymétrique ; et

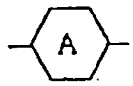

représente un groupe choisi parmi

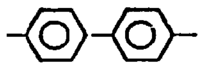 , 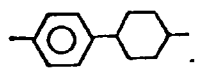 et 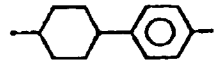 .

**2.** Composé d'ester d'acide carboxylique représenté par la formule [II] :

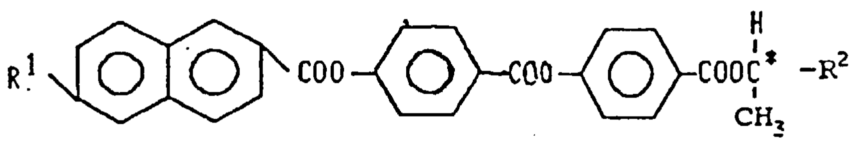 [II]

dans laquelle $R^1$ représente un groupe alkyle comportant de 3 à 20 atomes de carbone, un groupe alcoxy comportant de 3 à 20 atomes de carbone ou un groupe alkyle halogéné comportant de 3 à 20 atomes de carbone ; C* est un atome de carbone asymétrique ; et $R^2$ représente un groupe alkyle comportant de 2 à 10 atomes de carbone.

**3.** Utilisation d'un composé de formule [I] ou [II] telle que définies dans la revendication 1 ou 2, en tant que composé de cristaux liquides.

**4.** Composition de cristaux liquides comprenant un composé de formule [I] ou [II] telle que définie dans la revendication 1 ou 2.

**5.** Elément à cristaux liquides comprenant une cellule et deux plaques de polarisation, une sur chaque côté externe de la cellule, ladite cellule étant composée de deux substrats transparents disposés de manière à se faire face l'un à l'autre en laissant un intervalle entre les deux, et d'une électrode transparente appliquée sur la surface interne de chaque substrat, ledit intervalle étant rempli avec un matériau de cristaux liquides, dans lequel :

lesdites plaques de polarisation au nombre de deux sont disposées de manière à ce que l'angle formé par les plans de polarisation des plaques de polarisation soit compris entre 70 et 110°, et ladite cellule remplie avec le matériau de cristaux liquides est disposée entre les plaques de polarisation en un angle de +10° à -10° par rapport à la position de la cellule à laquelle la lumière transmise devient la plus sombre ou la plus brillante ; et

ledit matériau de cristaux liquides comprenant un composé de cristaux liquides représenté par la formule [III] :

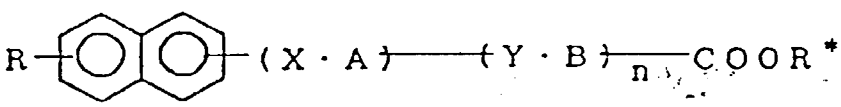 [III]

dans laquelle R représente un groupe alkyle comportant de 3 à 20 atomes de carbone, un groupe alcoxy comportant de 3 à 20 atomes de carbone ou un groupe alkyle halogéné comportant de 3 à 20 atomes de carbone ; X et Y représentent chacun un groupe choisi parmi -COO-, -OCO-, $-CH_2CH_2-$, $-CH_2O-$, $-O-CH_2-$,

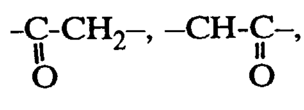

et -S-S- ou une liaison simple ;
R* représente un groupe choisi parmi

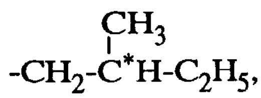, 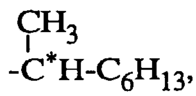, 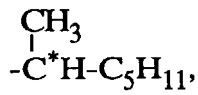, 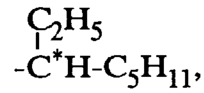, 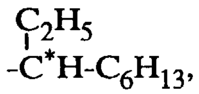, 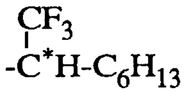 et 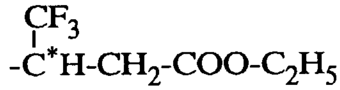 ;

n est un nombre entier de 1 ou 0 ; et quand n = 0, A représente

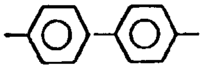 , 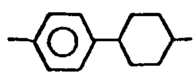 ou 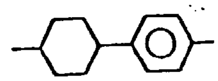

et quand n = 1, A et B représente chacun

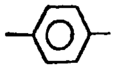 , 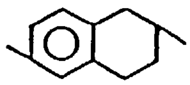 , 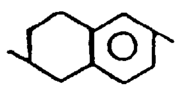 ou 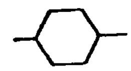 .

**6.** Elément conforme à la revendication 5, dans lequel le composé de formule [III] est un composé conforme à la revendication 1 ou 2.

**7.** Procédé de modulation de la lumière qui comprend le fait d'appliquer un champ électrique à un élément à cristaux liquides conforme à la revendication 5 ou 6.

**8.** Procédé conforme à la revendication 7, dans lequel la quantité de la lumière transmise par l'élément à cristaux liquides est changée en faisant varier la tension appliquée à l'élément entre une tension positive et une tension négative.

**9.** Procédé conforme à la revendication 7, dans lequel la quantité de lumière transmise par l'élément à cristaux liquides est changée en faisant varier la tension appliquée à l'élément à cristaux liquides entre 0 et une tension positive, ou entre 0 et une tension négative.

**10.** Procédé conforme à la revendication 8 ou 9, dans lequel le champ électrique se présente sous la forme d'onde rectangulaire, sous la forme d'onde triangulaire, sous la forme d'onde sinusoidale ou sous la forme d'une combinaison de telles ondes.

**11.** Procédé conforme à la revendication 10, dans lequel le champ électrique se présente sous la forme d'onde rectangulaire, dans lequel la largeur minimum de l'onde rectangulaire ne dépasse pas 10 ms.

**12.** Elément d'affichage utilisant un élément à cristaux liquides conforme à la revendication 5 ou 6.

**13.** Procédé d'affichage utilisant un procédé de modulation de la lumière conforme à l'une quelconque des revendications 7 à 11.

# FIG.1

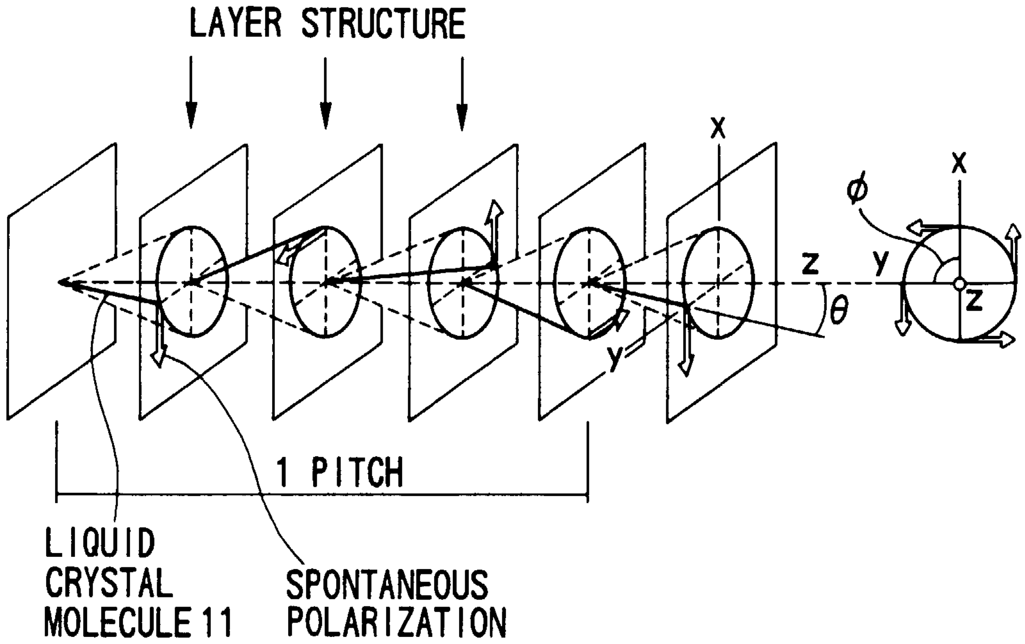
LAYER STRUCTURE
x
x
φ
z
y
θ
y
z
1 PITCH
LIQUID
CRYSTAL
MOLECULE 11
SPONTANEOUS
POLARIZATION

# FIG.2

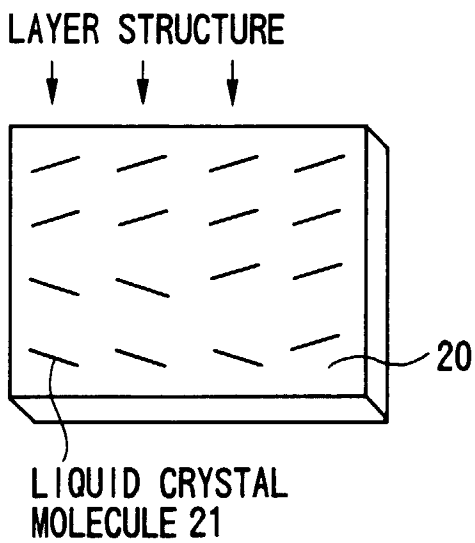
LAYER STRUCTURE
20
LIQUID CRYSTAL
MOLECULE 21

FIG.3

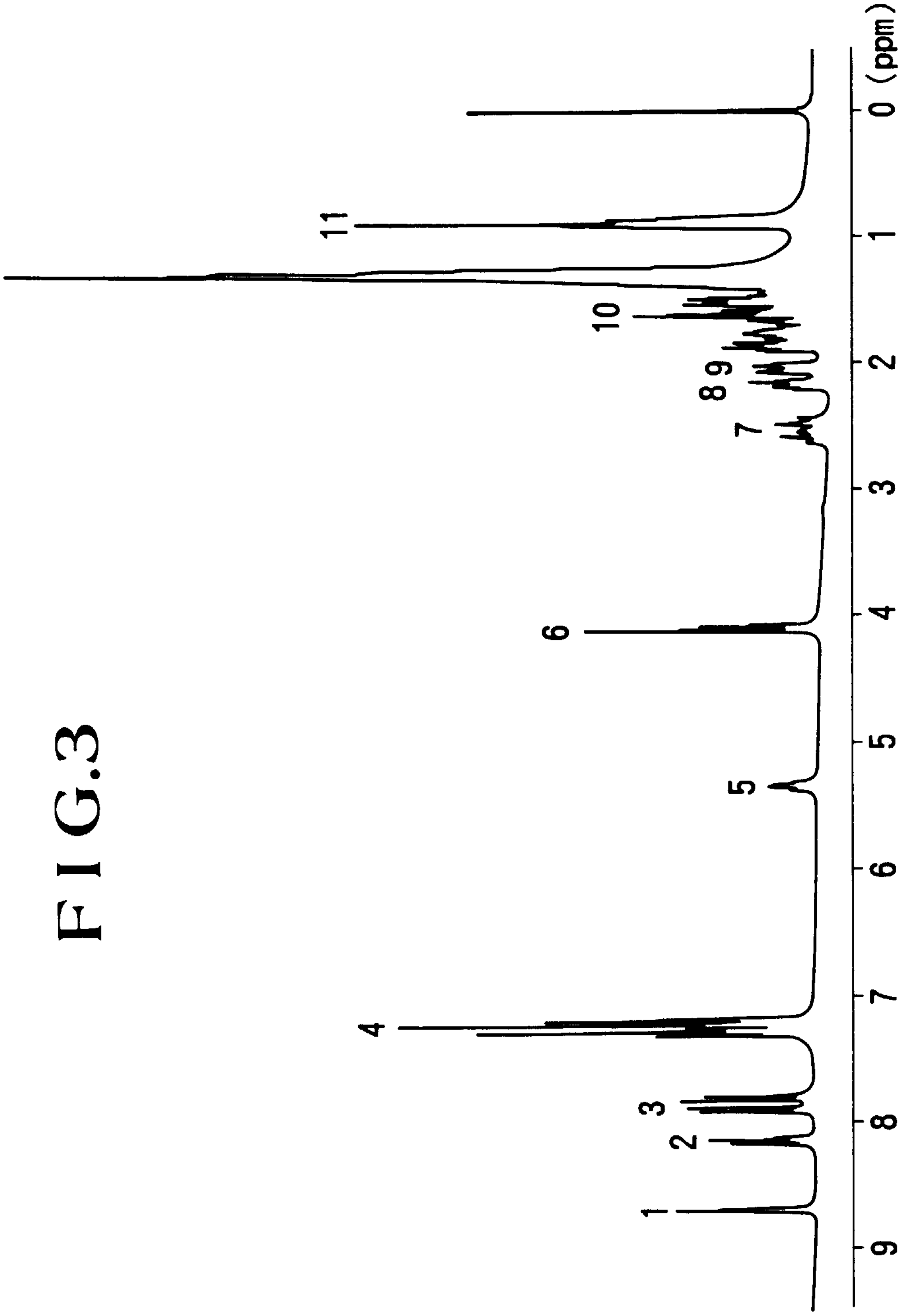

F I G. 4

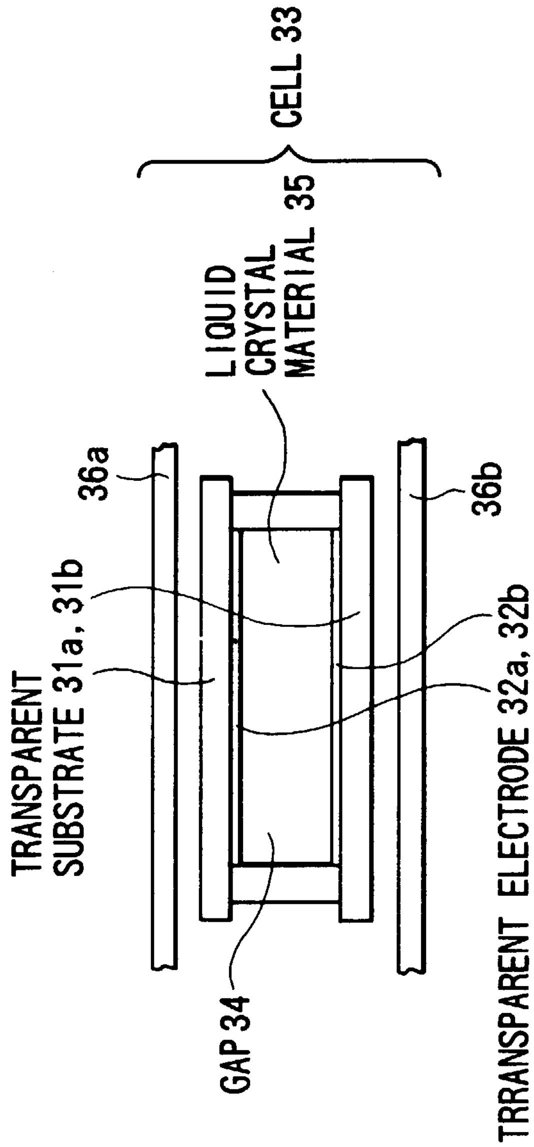
CELL 33
LIQUID CRYSTAL MATERIAL 35
36a
36b
TRANSPARENT SUBSTRATE 31a, 31b
TRRANSPARENT ELECTRODE 32a, 32b
GAP 34

FIG.5

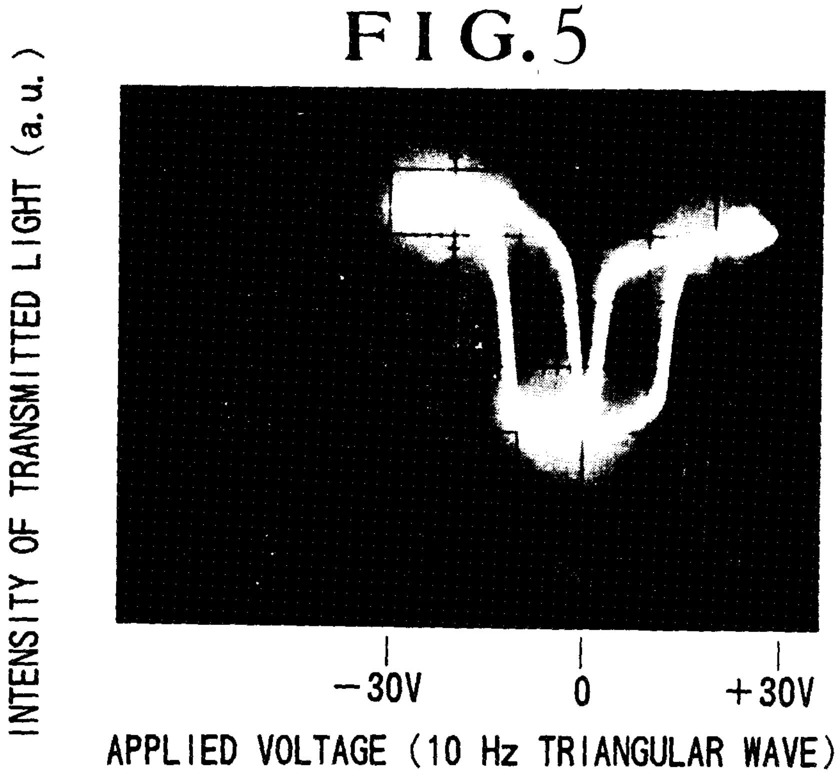
INTENSITY OF TRANSMITTED LIGHT (a.u.)
−30V
0
+30V
APPLIED VOLTAGE (10 Hz TRIANGULAR WAVE)

FIG.6

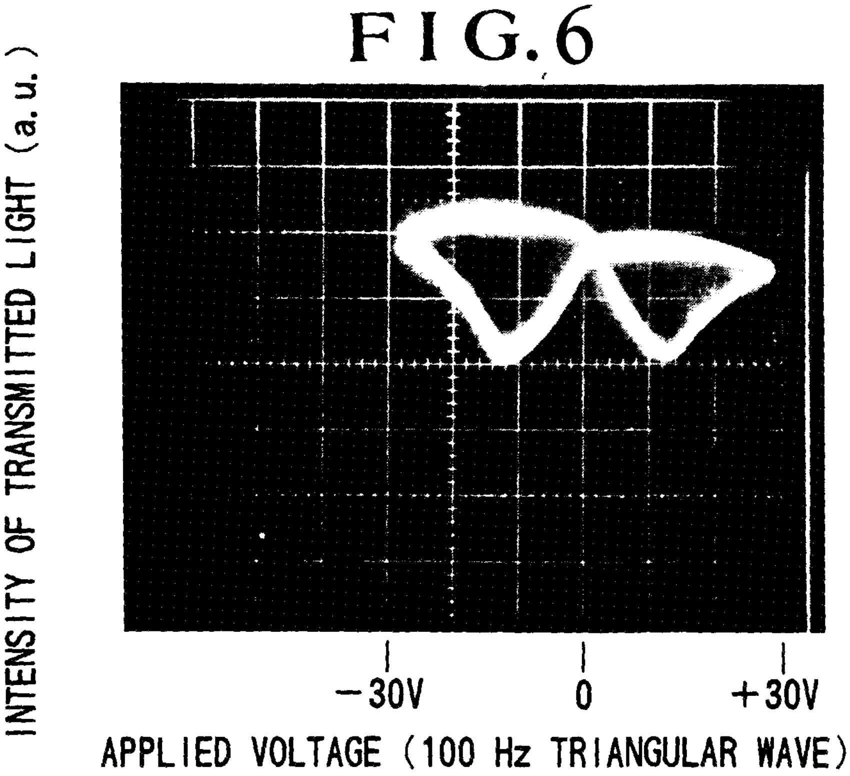
INTENSITY OF TRANSMITTED LIGHT (a.u.)
−30V
0
+30V
APPLIED VOLTAGE (100 Hz TRIANGULAR WAVE)

# FIG.7

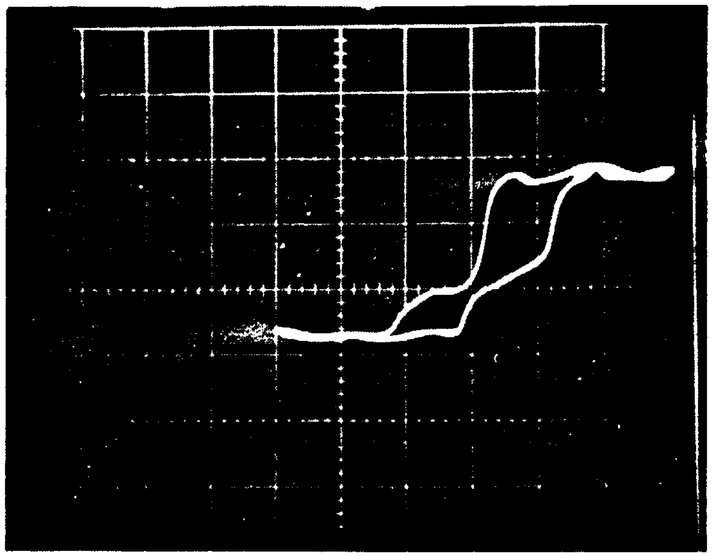
INTENSITY OF TRANSMITTED LIGHT (a.u.)
-30V
0
+30V
APPLIED VOLTAGE (10 Hz TRIANGULAR WAVE)

# FIG.8

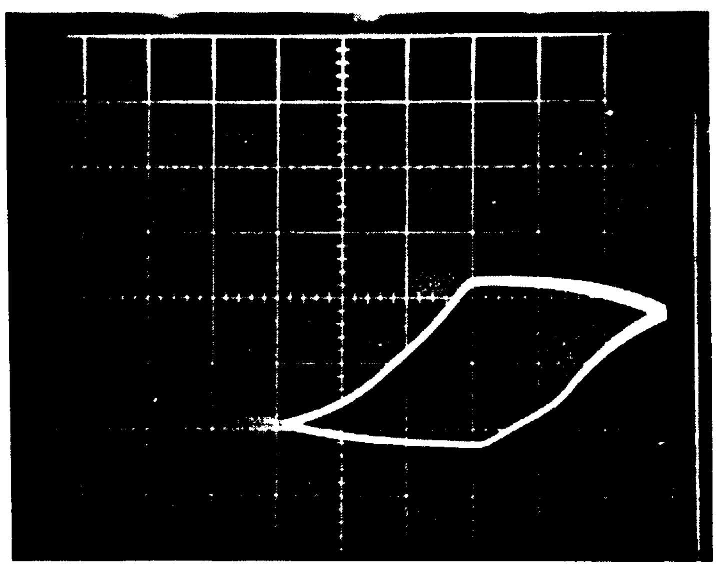
INTENSITY OF TRANSMITTED LIGHT (a.u.)
-30V
0
+30V
APPLIED VOLTAGE (100 Hz TRIANGULAR WAVE)